# EUROPEAN PATENT SPECIFICATION

(11) **EP 2 115 140 B1**
(45) Date of publication and mention of the grant of the patent: **30.11.2016**
(21) Application number: 07705747.9
(22) Date of filing: 31.01.2007
(51) Int. Cl.: C07D 471/04, A61K 31/437, A61P 31/18, A61P 35/00, C12N 15/29, C12N 15/09, C12N 15/10

(54) **Positively charged water-soluble prodrugs of 1H-imidazo[4,5-c]quinolin-4-amines and related compounds with very high skin penetration rates**
Positiv geladene wasserlösliche Arzneistoffvorstufen von 1H-Imidazo-[4,5-c]-chinolin-4-aminen und verwandten Verbindungen mit sehr hohen Hautdurchdringungsgeschwindigkeiten
Promédicaments hydrosolubles à charge positive de 1H-imidazo[4,5-c]quinolin-4-amines et composés associés a taux éléves de pénétration cutanée

(43) Date of publication of application: 11.11.2009
(73) Proprietor: Yu, Chongxi, Plainfield, Illinois 60585 (US)
(72) Inventor: Yu, Chongxi, Plainfield, Illinois 60585 (US)
(74) Representative: Finnegan Europe LLP
(86) International application number: PCT/IB2007/050322
(87) International publication number: WO 2008/093173

(56) References cited:
- US-A- 4 689 338
- US-A- 4 698 348
- GOBLYOS A. ET AL.: 'Structure-Activity Relationships of New 1H-Imidazo[4,5-c]quinolin-4-amines Derivatives as Allosteric Enhancers of the A3 Adenosine Receptor' J. MED. CHEM. vol. 49, 15 April 2006, pages 3354 - 3361, XP008115094
- JOHN F.G. ET AL.: 'Structure-Activity Relationships of 1H-Imidazo[4,5-c]quinolines That Induce Interferon Production' J. MED. CHEM. vol. 48, 21 April 2005, pages 3481 - 3491, XP008115095
- MARK J.B. ET AL.: '(1H-Imidazo[4,5-c]pyridin-2-yl)-1,2,5-oxad iazol-3-ylamine derivatives: Further optimization as highly potent and selective MSK-1-inhibitors' BIOORGANIC & MEDICINAL CHEMISTRY LETTERS vol. 15, 20 May 2005, pages 3407 - 3411, XP025314186

## Description

### Technical Field

The present invention relates to the preparations of positively charged and water- soluble pro-drugs of 1H-imidazo[4, 5-c]quinolin-4-amines and related compounds and their medicinal use in treating conditions in humans or animals. More specifically, the present invention is to enable quick skin penetration of 1H-imidazo[4, 5-c]quinolin-4-amines and related compounds.

### Background Art

1H-Imidazo[4, 5-c]quinolin-4-amines and related compounds are disclosed in U.S. Pat. No. 4,689,338 and described therein as antiviral agents and as an interferon inducer. The anti-herpes activity of 1-Isobutyl-1H-imidazo[4, 5-c]quinolin-4-amine and related compounds relative to primary lesions caused by the herpes simplex virus is demonstrated by Gerster, J.F. (U.S. Pat. No. 4,689,338) using the method described generally by Kern, et al. (Antimicrob. Agents Chemother. 14, 817-823, 1978).

A variety of formulations for topical administration of this compound is also described. U.S. Pat. No. 4,751,087, 4,411,893, 4,722,941, 4,746,515, and 5,736,553 disclosed the use of a combination of ethyl cleats and glyceryl monolaurate, N,N-dimethyldodecylamine-N-oxide, glyceryl monolaurate, and fatty acid as skin penetration enhancers to enhance the transdermal flux of drugs. Aldara (1-Isobutyl-1H-imidazo[4, 5-c]quinolin-4-amine) cream has been developed by 3M for the treatment of actinic keratosis, superficial basal cell carcinoma, and external genital and perianal warts. Göblyös et al. (Göblyös. A et al. (2006) J Med Chem. 49. 3354-3361) discloses 1H-imidazo[4,5-c]quinolin-4-amines derivatives as allosteric enhancers of the A3 adenosine receptor. Gerster et al. (Gerster, JF et al. (2005) J Med Chem, 48, 3481-3491) disclose that 1H-imidazo[4, 5-c]quinolines induce iterferon production.

### Disclosure of Invention

### Technical Problem

Aldara (1-Isobutyl-1H-imidazo[4, 5-c]quinolin-4-amine) cream has been developed by 3M for the treatment of actinic keratosis, superficial basal cell carcinoma, and external genital and perianal warts.

Unfortunately , 1-Isobutyl-1H-imidazo[4, 5-c]quinolin-4-amine and related compounds have very low solubility in water and organic solvents so their skin penetration rates are very low. The cream compositions will keep the drug on the skin for a very long time in very high concentration and cause many side effects that include redness, swelling, sores, blisters, or ulcers, skin that becomes hard or thickened, skin peeling, scabbing and crusting, itching, burning and changes in skin color that do not always go away. Another problem is that they cannot penetrate skin deep enough to treat cancers except for superficial basal cell carcinoma.

### Technical Solution

This invention relates to the preparation of novel positively charged pro-drugs of 1H-imidazo[4, 5-c]quinolin-4-amines and related compounds and their medicinal use. The pro-drugs of 1 H-imidazo[4, 5-c]quinolin-4-amines and related compounds have the general formula (1) 'Structure 1' or formula (2) 'Structure 2'. The present invention provides a compound having a general formula of Structure 1 or Structure 2, 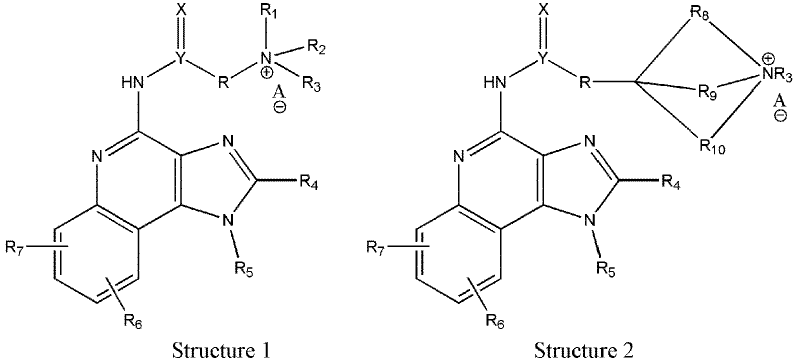 wherein,
R represents a branched or straight chain, -(CH₂)ₙ-, wherein n= 0, 1, 2, 3, 4, 5, 6, 7, 8, 9, 10, in -(CH₂)ₙ-, any CH₂ may be replaced with O, S, CH=CH, C=C, or CR₁₁R₁₂;
R₁ and R₂ are independently selected from the group consisting of H, alkyl having 1 to 15 carbon atoms, alkyloxy having 1 to 15 carbon atoms, alkenyl having up to 15 carbon atoms, perfluoroalkyl having 1 to 15 carbon atoms, alkyl halide having 1 to 15 carbon atoms, alkynyl having up to 15 carbon atoms, aryl, and heteroaryl moieties, or taken together to -(CH₂)ₘ-, wherein m= 2, 3, 4, 5, 6, 7, 8, 9, 10, and any CH₂ may be replaced with O, S, CH=CH, C=C;
R₃ is selected from the group consisting of H, alkyl having 1 to 15 carbon atoms, alkyloxy having 1 to 15 carbon atoms, alkenyl having up to 15 carbon atoms, perfluoroalkyl having 1 to 15 carbon atoms, alkyl halide having 1 to 15 carbon atoms, alkynyl having up to 15 carbon atoms, aryl and heteroaryl moieties, wherein, any CH₂ may be replaced with O, S, CH=CH, C=C, or CR₁₁R₁₂;
X represents O, NH, or S;
Y represents C, S=O, or P-OR₁₁;
R₄ is selected from the group consisting of alkyl of 1 to 10 carbon atoms, hydroxylalkyl of 1 to 10 carbon atoms, acyloxyalkyl wherein the acyloxy moiety is alkanoyloxy of 2 to 5 carbon atoms or benzoyloxy, and the alkyl moiety contains 1 to 8 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to 5 carbon atoms, alkoxy of 1 to 5 carbon atoms and halogen, with the proviso that if said benzene ring is substituted by 2 of said moieties, then said moieties together contain no more than 8 carbon atoms;
R₅ is selected from the group consisting of hydrogen, alkyl of 1 to 10 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to 5 carbon atoms, alkoxy of 1 to 5 carbon atoms and halogen, with the proviso that if said benzene ring is substituted by 2 of said moieties, then said moieties together contain no more than 8 carbon atoms;
R₆ is selected from the group consisting of hydrogen, alkyl of 1 to 5 carbon atoms, and alkoxy of 1 to 5 carbon atoms and halogen;
R₇ is selected from the group consisting of hydrogen, alkyl of 1 to 5 carbon atoms, and alkoxy of 1 to 5 carbon atoms and halogen;
R₈ represents a branched or straight chain, -(CH₂)ₙ-, wherein a= 0, 1, 2, 3, 4, 5, 6, 7, 8, 9, 10, in -(CH₂)ₙ-, any CH₂ may be replaced with O, S, CH=CH, C=C, or CR₁₁R₁₂;
R₉ represents a branched or straight chain, -(CH₂)_{b}-, wherein b= 0, 1, 2, 3, 4, 5, 6, 7, 8, 9, 10, in -(CH₂)_{b}-, any CH₂ may be replaced with O, S, CH=CH, C=C, or CR₁₁R₁₂;
R₁₀ represents a branched or straight chain, -(CH₂)_{c}-, wherein c= 0, 1, 2, 3, 4, 5, 6, 7, 8, 9, 10, in -(CH₂)_{c}-, any CH₂ may be replaced with O, S, CH=CH, C=C, or CR₁₁R₁₂;
R₁₁ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C=C, or CR₁R₂;
R₁₂ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C=C, or CR₁R₂;
A⁻ represents a negative ion;
and all R, R₁, R₂, R₃, R₄, , -(CH₂)ₙ-, -(CH₂)ₘ-, -(CH₂)ₐ-, -(CH₂)_{b}-, or -(CH₂)_{c}-groups are branched or straight chains and may include C, H, O, S, P, or N atoms and may have single, double, and triple bonds.

The present invention further provides a compound selected from the group consisting of sarcosine 1-isobutyl-1 H-imidazo[4, 5-c) quinolin-4-amide.HA, N,N-dimethylglycine 1-isobutyl-1 H-imidazo[4, 5-c]quinolin-4-amide.HA, sarcosine 1-butyl-1H-imidazo[4, 5-c]quinolin-4-amide.HA, sarcosine 1-benzyl-1 H-imidazo [4, 5-c]quinolin-4-amide.HA, sarcosine 1-methyl-H-imidazo [4, 5-c] quinolin-4-amide.HA, sarcosine 1, 2, 8-trimethyl-1 H-imidazo[4, 5-c]quinolin-4-amide.HA, sarcosine 1-(2-hydroxyethyl)-1H-imidazo[4,5-c]quinolin-4-amide.HA, sarcosine 1, 8-dimethyl-1H-imidazo[4, 5-c]quinolin-4-amide.HA, sarcosine 1, 2-dimethyl-1 H-imidazo[4, 5-c]quinolin-4-amide.HA, sarcosine 1-(2,3-dihydroxypropyl)-1H-imidazo[4, 5-c]quinolin-4-amide.HA, sarcosine 1-cyclohexylmethyl-1H-imidazo[4, 5-c]quinolin-4-amide.HA, sarcosine 1-benzyl-2-methyl-1 H-imidazo[4,5-c]quinolin-4-amide.HA, sarcosine 1-n-hexyl-1H-imidazo[4, 5-c]quinolin-4-amide.HA, and sarcosine 1-n-hexyl-2-methyl-1H-imidazo[4,5-c]quinolin-4-amide.HA, wherein, A⁻ represents a negative ion.

Drug absorption, whether from the gastrointestinal tract or other sites, requires the passage of the drug in a molecular form across the barrier membrane. The drug must first dissolve, and if the drug possesses the desirable biopharmaceutical properties, it will pass from a region of high concentration to a region of low concentration across the membrane into the blood or general circulation. All biological membranes contain lipids as major constituents. The molecules that play the dominant roles in membrane formation all have phosphate-containing highly polar head groups, and, in most cases, two highly hydrophobic hydrocarbon tails. The membranes are bilayers, with the hydrophilic head groups facing outward into the aqueous regions on either side. Very hydrophilic drugs cannot pass the hydrophobic layer of a membrane and very hydrophobic drugs will stay in the hydrophobic layer as part of the membrane due to their similarities and cannot efficiently enter the cytosol on the inside.

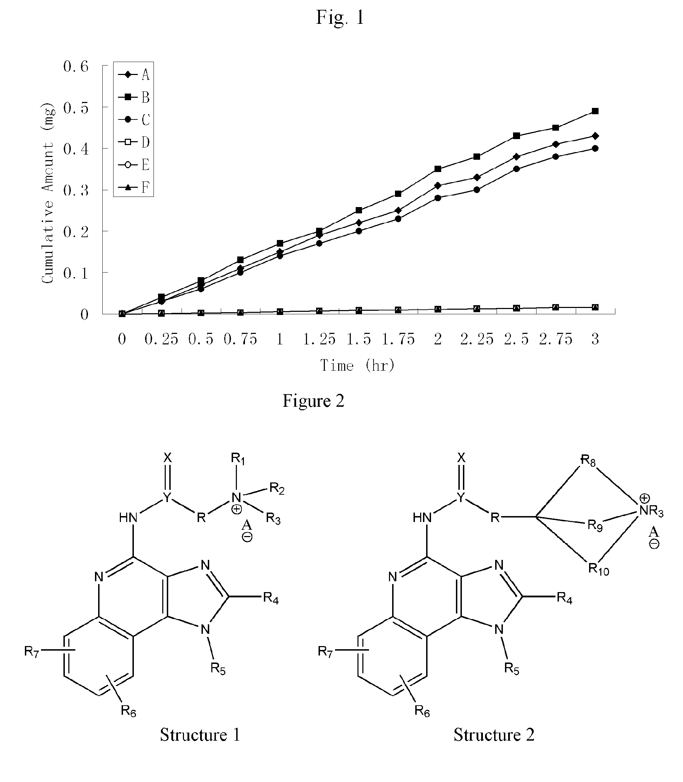

The goal of this invention is to make 1H-Imidazo[4, 5-c]quinolin-4-amines and related compounds administrable transdermally (topical application) by increasing their solubility in the moisture available on the skin surface and increasing their penetration rate through the membrane and skin barrier. These novel pro-drugs of 1H-Imidazo[4, 5-c]quinolin-4-amines and related compounds have two structural features in common: they have a lipophilic portion and a primary, secondary, or tertiary amine group that exists in the protonated form (hydrophilic part, their pKa are 9.5-10.7) at physiological pH [The aromatic amines of 1H-Imidazo[4, 5-c] quinolin-4-amines are very weak bases (pKa is 6.5-6.7) and only a small part of them exists in the protonated form at physiological pH]. Such a hydrophilic-lipophilic balance is required for efficient passage through the membrane barrier [Susan Milosovich, et al., J. Pharm. Sci., 82, 227(1993)]. The positively charged amino groups largely increase the solubility of the drugs in water. In many instances, the lowest or rate-limiting step in the sequence is the dissolution of the drug. 1H-Imidazo[4, 5-c]quinolin-4-amines and related compounds have a very low solubility in the moisture available on the skin surface, and they will not pass across the barrier of skin in a molecular form efficiently. Even if they enter the membranes of the skin, they cannot efficiently enter the cytosol, a semi-liquid concentrated aqueous solution or suspension on the inside of the cell (due to their low water solubility). When these new pro-drugs are administered transdermally in a dosage form such as a solution, spray, lotion, ointment, emulsion or gel, they will dissolve in the moisture available on the skin surface immediately. The positive charge on the amino groups of these pro-drugs will bond to the negative charge on the phosphate head group of a membrane [the bonding between the positive charge on the aromatic amines of 1H-imidazo[4, 5-c]quinolin-4-amines and the negative charge on the phosphate head group of a membrane is very weak due to the weak basity (pKa is about 6.5-6.7) and hindered position (the amino groups connected with aromatic ring directly)], thus, the local concentration of the outside of the membrane will be very high and will facilitate the passage of these pro-drugs from a region of high concentration to a region of low concentration. When these pro-drugs enter the membrane, the hydrophilic part will push the pro-drug into the cytosol. When the prodrugs are outside the skin membranes, they are only prodrugs, which may be without biological activity; because of that and due to the short stay on the outside of the membrane, the prodrugs will not cause redness, swelling, sores, blisters, or ulcers in the skin, hardened or thickened skin, skin peeling, scabbing and crusting, itching, burning or changes in skin color. The penetration rates of these prodrugs through human skin were measured in vitro by using modified Franz cells, which were isolated from human skin tissue (360-400 µm thick) of the anterior and posterior thigh areas. The receiving fluid consisted of 2 ml of 2% bovine serum albumin in normal saline and was stirred at 600 rpm. The cumulative amounts of these prodrugs and their parent drugs penetrating the skin versus time were determined by a specific high-performance liquid chromatography method. The results using a donor consisting of either a 3% solution of some of the prodrugs of 1H-imidazo[4, 5-c]quinolin-4-amines or a 3% suspension of 1H-imidazo[4, 5-c]quinolin-4-amines in 0.5mL of a mixture of ethanol and pH 7.4-phosphate buffer (0.2M) (v/v, 70/30) are shown in Figure 1. Apparent flux values of 0.15 mg, 0.13 mg, 0.16 mg, 0.005 mg, 0.005 mg, and 0.005 mg/cm²/h were calculated for sarcosine 1-isobutyl-1H-imidazo[4, 5-c]quinolin-4-amide hydrochloride, sarcosine 1-butyl-1H-imidazo[4, 5-c]quinolin-4-amide hydrochloride, sarcosine 1-benzyl-1H-imidazo[4, 5-c]quinolin-4-amide hydrochloride, 1-isobutyl-1H-imidazo[4, 5-c]quinolin-4-amide hydrochloride, 1-butyl-1H-imidazo[4, 5-c]quinolin-4-amine hydrochloride, and 1-benzyl-1H-imidazo[4, 5-c] quinolin-4-amine hydrochloride respectively diffusing through human skin. The pro-drugs diffuse through human skin more than 25 times faster than do 1H-imidazo[4, 5-c] quinolin-4-amines. The results suggest that the positive charge on the di-alkyaminoethyl group has a very important role in the passage of the drug across the membrane and skin barrier.

Irritative effect or discomfort in the skin of mice of the novel prodrugs was evaluated during a period of 1 week after the topical application of 0.05 ml of 3 % of the respective test drug in pH 7.4 phosphate buffer (0.2 M) to the back of nude mice twice per day. None of any signs of irritative effect or discomfort was observed for sarcosine 1-isobutyl-1H-imidazo[4, 5-c]quinolin-4-amide hydrochloride, sarcosine 1-butyl-1H-imidazo[4, 5-c]quinolin-4-amide hydrochloride, sarcosine 1-benzyl-1H-imidazo[4, 5-c]quinolin-4-amide hydrochloride.

A good prodrug should change back to the parent drug easily. In vitro plasma hydrolysis studies were carried out as the following. 10 mg of the prodrug was dissolved in 0.1 ml of 0.2M pH 7.4 phosphate buffer. 1 ml of human plasma, preheated to 37°C, was added into the mixture. The mixture was kept in a water bath at 37°C. At every 2 min. intervals, 0.2 ml of samples were withdrawn and added to 0.4 ml of methanol to precipitate the plasma protein. The samples were centrifuged for 5 min and analyzed by HPLC. The half lives of hydrolysis are 18 min+/- 1 min. for sarcosine 1-isobutyl-1H-imidazo[4, 5-c]quinolin-4-amide hydrochloride, 18 min+/- 2 min. for sarcosine 1-butyl-1H-imidazo[4, 5-c]quinolin-4-amide hydrochloride, 19 min +/-1 min. for sarcosine 1-isobutyl-1H-imidazo[4, 5-c]quinolin-4-amide hydrochloride

1H-imidazo[4, 5-c]quinolin-4-amines are known antiviral agents that are also known to induce interferon biosynthesis (Gerster, J.F., U.S. Pat. No. 4,689,338). The fact that the compounds are interferon inducers suggests that they may be useful in the treatment of numerous diseases, such as rheumatoid arthritis, genital or other warts, eczema, hepatitis, psoriasis, multiple sclerosis, essential thrombocythaemia, cancers, acquired immunodeficiency syndrome (AIDS) and other viral diseases. Aldara (1-Isobutyl-1H-imidazo[4, 5-c]quinolin-4-amine) cream has been developed by 3M for the treatment of actinic keratosis, superficial basal cell carcinoma, and external genital and perianal warts.

For evaluation of antitumor activity of these prodrugs, human breast cancer cells (BCAP-37, 4-5 mm³ of tumor tissue was used in each mouse) were subcutaneously xenografted into nude mice (BALB). After 3 hour, 50 µl of 3 % of sarcosine 1-isobutyl-1H-imidazo[4, 5-c]quinolin-4-amide hydrochloride and 1-isobutyl-1H-imidazo[4, 5-c]quinolin-4-amide hydrochloride in ethano1/0.2M pH 7.2 phosphate buffer (v/v, 70/30) was topically applied to the human breast cancer cells-implanted area (near the front leg) once per day. After 28 days, the control group (n=7, the average tumor size was 15 ± 2 mm x 13 ± 2 mm) and the groups that were treated with 1-isobutyl-1H-imidazo[4, 5-c]quinolin-4-amide hydrochloride group (n=7, the average tumor size was 12 ± 2 mm x 11 ± 2 mm) demonstrated 100% incidence , but none of tumor was seen in the group (n=7) that were treated with sarcosine 1-isobutyl-1H-imidazo[4, 5-c]quinolin-4-amide hydrochloride. The average weights of the mice were 22 ± 2 grams for the sarcosine 1-isobutyl-1H-imidazo[4, 5-c] quinolin-4-amide hydrochloride treated group, 22 ± 2 grams for 1-isobutyl-1H-imidazo[4, 5-c] quinolin-4-amide hydrochloride treated group , and 24 ± 2 grams for the control group. The results also show that the prodrug has very mild side effects.

In the second antitumor experiment, human colon cancer cells (LS174J, 4-5 mm³ of tumor tissue was used in each mouse) were subcutaneously xenografted into nude mice (BALB). After 3 hour, 50 µl of 3 % of sarcosine 1-isobutyl-1H-imidazo[4, 5-c] quinolin-4-amide hydrochloride and 1-isobutyl-1H-imidazo[4, 5-c]quinolin-4-amide hydrochloride in ethanol/0.2M pH 7.2 phosphate buffer (v/v, 70/30) was topically applied to the human colon cancer cells-implanted area (near the front leg) (twice per day). After 28 days, the control group (n=7, the average tumor size was 20 ± 3 mm x 18 ± 3 mm) and the groups that were treated with 1-isobutyl-1H-imidazo[4, 5-c] quinolin-4-amide hydrochloride group (n=7, the average tumor size was 17 ± 2 mm x 15 ± 2 mm) demonstrated 100% incidence , but none of tumor was seen in the group (n=7) that were treated with sarcosine 1-isobutyl-1H-imidazo[4, 5-c]quinolin-4-amide hydrochloride. The average weights of the mice are 21 ± 2 grams for the sarcosine 1-isobutyl-1H-imidazo[4, 5-c]quinolin-4-amide hydrochloride treated group, 21 ± 2 grams for 1-isobutyl-1H-imidazo[4, 5-c]quinolin-4-amide hydrochloride treated group , and 23 ± 2 grams for the control group.

In the third antitumor experiment, human breast cancer cells (BCAP-37, 3-4 mm³ of tumor tissue was used in each mouse) were subcutaneously xenografted into nude mice (BALB). After 21 days, the tumors were growing to the size of 13 ± 2 mm x 12 ± 3 mm. Then 50 µl of 3 % of sarcosine 1-isobutyl-1H-imidazo[4, 5-c]quinolin-4-amide hydrochloride and 1-isobutyl-1H-imidazo[4, 5-c]quinolin-4-amide hydrochloride in ethanol/0.2M pH 7.2 phosphate buffer (v/v, 70/30) was topically applied to the human breast cancer cells-implanted area (near the front leg) (twice per day). The tumors are growing to the size of 21 ± 3 mm x 19 ± 3 mm in the control group (n=7) at day 45 and all mice died by the 60^{th} day. In the group (n=7) that was treated with 1-isobutyl-1H-imidazo[4, 5-c]quinolin-4-amide hydrochloride , the tumors were 17 ± 2 mm x 15 ± 2 mm at day 45 and all mice died by the day 70. The tumors in the group (n=7) that were treated with sarcosine 1-isobutyl-1H-imidazo[4, 5-c]quinolin-4-amide hydrochloride had shrunk to 11 ± 2 mm x 10 ± 2 mm at day 45 and none of mice died at the day 70. The average weights of the mice were 21 ± 2 grams for the sarcosine 1-isobutyl-1H-imidazo[4, 5-c]quinolin-4-amide hydrochloride treated group, 22 ± 2 grams for 1-isobutyl-1H-imidazo[4, 5-c]quinolin-4-amide hydrochloride treated group , and 23 ± 2 grams for the control group at day 45.

The evaluation of anti-herpes activity of these prodrugs was carried out using the method described by Kern, et al. [Antimicrob. Agents Chemother. 14, 817, (1978)]. In the first experiment, female guinea pigs (n=5) were anesthetized. About 100 µl of 3% of sarcosine 1-isobutyl-1H-imidazo[4, 5-c]quinolin-4-amide hydrochloride in ethanol/ 0.2M pH 7.4 phosphate buffer (v/v, 70/30) was applied intravaginally to the guinea pigs for 3 days. The guinea pigs were then infected with herpes simplex virus (Type I or Type II, about 10⁵ plaque forming units were used) intravaginally using a cotton swab. Virus replication was monitored by determining the amount of virus recovered with vaginal swabs taken on days 1, 2, 3, 5, or 7 after infection. In the second experiment, the female guinea pigs (n=5) were anesthetized. About 200 µl of 3% of sarcosine 1-isobutyl-1H-imidazo[4, 5-c]quinolin-4-amide hydrochloride in ethanol/ 0.2M pH 7.4 phosphate buffer (v/v, 70/30) was applied intravaginally to the guinea pigs and herpes simplex virus (Type I or Type II, about 10⁵ plaque forming units were used) was applied intravaginally to the same guinea pigs using a cotton swab. In the third experiment, the female guinea pigs (n=5) were anesthetized. The guinea pigs were then infected with herpes simplex virus (type I or Type II, about 10⁵ plaque forming units were used) intravaginally using a cotton swab. After 5 days, about 100 µl of 5% of sarcosine 1-isobutyl-1H-imidazo[4, 5-c]quinolin-4-amide hydrochloride in ethanol/0.2M pH 7.4 phosphate buffer (v/v, 70/30) was applied intravaginally once per day for 10 days. It has been found that the compound sarcosine 1-isobutyl-1H-imidazo[4, 5-c]quinolin-4-amide hydrochloride is effective when administered to guinea pigs beginning before, same time, or after infection.

1H-imidazo[4, 5-c]quinolin-4-amines can be prepared using the methods disclosed in U.S. Pat. No. 4, 689, 338. The compounds of the general formula (1)'Structure 1' or formula (2) 'Structure 2' indicated above can be prepared from 1H-imidazo[4, 5-c] quinolin-4-amines or related compounds, by reaction with compounds of the general formula (3) 'Structure 3' by using coupling reagents, such as N,N'-Dicyclohexylcarbodiimide, N, N'-Diisopropylcarbodiimide, O- (Benzotriazol- 1 -yl)-N,N,N',N'-tetramethyluronium tetrafluoroborate, O- (Benzotriazol- 1 -yl)-N,N,N',N'-tetramethyluronium hexafluorophosphate, Benzotriazol- 1-yl-oxy-tris(dimethylamino)phosphonium hexafluorophosphate, 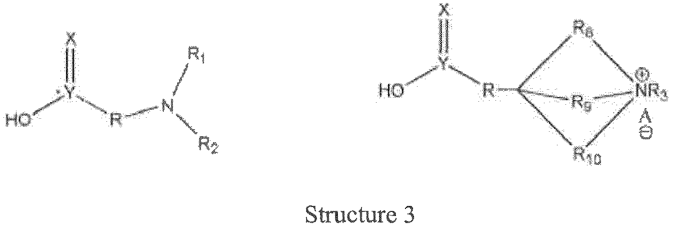 wherein, R represents a branched or straight chain, -(CH₂)ₙ-, wherein n=0, 1, 2, 3, 4, 5, 6, 7, 8, 9, 10, in -(CH₂)ₙ-, any CH₂ may be replaced with O, S, CH=CH, C≡C, CR₁₁R₁₂, aryl or heteroaryl residues; R₁ and R₂ are independently selected from the group consisting of H, one of any alkyl, alkyloxy, alkenyl or alkynyl residues having up to 15 carbon atoms, aryl or heteroaryl moieties or taken together are -(CH₂)ₘ-, wherein m=2, 3, 4, 5, 6, 7, 8, 9, 10, and any CH₂ may be replaced with O, S, CH=CH, C≡C, CR₁₁R₁₂, aryl or heteroaryl moieties; X represents O, NH, or S; Y represents C, S=O, or P-OR₁₁; R₃₄ represents H, one of any alkyl, alkyloxyl, alkenyl or alkynyl residues having up to 15 carbon atoms, aryl or heteroaryl moieties; R₈ represents a branched or straight chain, -(CH₂)ₐ-, wherein a=0, 1, 2, 3, 4, 5, 6, 7, 8, 9, 10, in -(CH₂)ₐ-, any CH₂ may be replaced with O, S, CH=CH, C≡C, CR₁₁R₁₂, aryl or heteroaryl residues; R₉ represents a branched or straight chain, -(CH₂)_{b}-, wherein b=0, 1, 2, 3, 4, 5, 6, 7, 8, 9, 10, in -(CH₂)_{b}-, any CH₂ may be replaced with O, S, CH=CH, C≡C, CR₁₁R₁₂, aryl or heteroaryl residues ; R₁₀ represents a branched or straight chain, -(CH₂)_{c}-, wherein c=0, 1, 2, 3, 4, 5, 6, 7, 8, 9, 10, in -(CH₂)-, any CH₂ may be replaced with O, S, CH=CH, C≡C, CR₁₁R₁₂, aryl or heteroaryl residues; R₁₁ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C≡C, CR₁₁R₁₂, aryl or heteroaryl moieties; R₁₂ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C≡C, CR₁₁R₁₂, aryl or heteroaryl moieties; A⁻ represents a negative ions. All R, R₁, R₂, R₃, R₄, -(CH₂)ₙ-, -(CH₂)ₘ-, -(CH₂)ₐ-, -(CH₂)_{b}-, or -(CH₂)_{c}- groups are branched or straight chains and may include C, H, O, S, P, or N atoms and may have single, double, and triple bonds.

Further herein described is that the compounds of the general formula (1) 'Structure 1' or formula (2) 'Structure 2' indicated above can be prepared from 1H-imidazo[4, 5-c]quinolin-4-amines and related compounds, by reaction with compounds of the general formula (4) 'Structure 4', 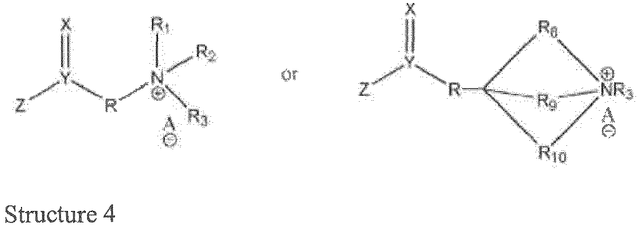 wherein, R represents a branched or straight chain, -(CH₂)ₙ-, wherein n=0, 1, 2, 3, 4, 5, 6, 7, 8, 9, 10 ..., and any CH₂ may be replaced with O, S, CH=CH, C≡C, CR₁₁R₁₂, aryl or heteroaryl residues, or other ring systems; R₁ and R₂ taken alone are same or different and are H, one of any alkyl, alkyloxyl, alkenyl, perfluoroalkyl, alkyl halide or alkynyl residues having 1 to 12 carbon atoms, aryl or heteroaryl moieties or taken together are -(CH₂)ₘ-, wherein m=2, 3, 4, 5, 6, 7, 8, 9, 10,..., and any CH₂ may be replaced with O, S, CH=CH, C≡C, CR₁₁R₁₂ aryl or heteroaryl moieties, or other ring moieties; R₃ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with 0, S, CH=CH, C≡C, CR₁₁R₁₂, aryl or heteroaryl moieties, or other ring moieties; R₈ represents a branched or straight chain, -(CH₂)ₐ-, wherein a=0, 1, 2, 3, 4, 5, 6, 7, 8, 9, 10 ..., in -(CH₂)ₐ-, any CH may be replaced with O, S, CH=CH, C≡C, CR₁₁R₁₂, aryl or heteroaryl residues, or other ring systems; R₉ represents a branched or straight chain, -(CH₂)_{b}-, wherein b=0, 1, 2, 3, 4, 5, 6, 7, 8, 9, 10 ..., in -(CH₂)_{b}-, any CH₂ may be replaced with O, S, CH=CH, C≡C, CR₁₁R₁₂, aryl or heteroaryl residues, or other ring systems; R₁₀ represents a branched or straight chain, -(CH₂)_{c}-, wherein c=0, 1, 2, 3, 4, 5, 6, 7, 8, 9, 10 ..., in -(CH₂)_{c}-, any CH may be replaced with O, S, CH=CH, C≡C, CR₁₁R₁₂, aryl or heteroaryl residues, or other ring systems; R represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR₁ R₂, aryl or heteroaryl moieties, or other ring moieties; R₁₂ represents H, one of any alkyl, alkyloxy, alkenyl, perfluoroalkyl, alkyl halide, or alkynyl residues having 1 to 12 carbon atoms, aryl or heteroaryl moieties, wherein, any CH₂ may be replaced with O, S, CH=CH, C≡C, CR R , aryl or heteroaryl moieties, or other ring moieties; A⁻ represents Cl⁻, Br⁻ F, I⁻, AcO⁻, citrate, or any negative ions. All R, R , R , R , R , -(CH₂ )ₙ-, -(CH₂)ₘ-, -(CH₂)ₐ -, -(CH₂)_{b}-, or -(CH₂)_{c}- groups are branched or straight chains and may include C, H, O, S, P, or N atoms and may have single, double, and triple bonds.

### Advantageous Effects

These pro-drugs of 1H-imidazo[4, 5-c]quinolin-4-amines and related compounds in the present invention have a lipophilic portion and a hydrophilic portion (the amine groups that exist in the protonated form at physiological pH). The positively charged amino groups of these pro-drugs have two major advantages. First, it largely increases the solubility of the drugs in water; when these new pro-drugs are administered transdermally in a dosage form such as a solution, spray, lotion, ointment, emulsion or gel, they will mix with the moisture on the skin, eye, genital area, mouth, nose, or other part of the body immediately. Second, the positive charge on the amino group of these pro-drugs will bond to the negative charge on the phosphate head group of the membrane. Thus, the local concentration outside of the membrane will be very high and will facilitate the passage of these pro-drugs from a region of high concentration to a region of low concentration. When these pro-drugs enter the membrane, the hydrophilic part will push the pro-drugs into the cytosol, a semi-liquid concentrated aqueous solution or suspension. Due to the short stay on the skin, eye, genital area, mouth, nose, or other part of the body, the pro-drugs will not cause itching, burning, or pain. Experiment results show that more than 90% of the pro-drugs were changed back to the parent drugs in a few minutes. The pro-drugs have a much better absorption rate and as transdermal administration avoids the first pass metabolism, the pro-drugs will be stronger than 1H-imidazo[4, 5-c]quinolin-4-amines and related compounds at the same dosage.

### Description of Drawings

Figure 1: Cumulative amounts of sarcosine 1-isobutyl-1H-imidazo[4, 5-c] quinolin-4-amide hydrochloride (3% solution, A), sarcosine 1-butyl-1H-imidazo[4, 5-c] quinolin-4-amide hydrochloride (3% solution, B), sarcosine 1-benzyl-1H-imidazo[4, 5-c]quinolin-4-amide hydrochloride (3% solution, C), 1-isobutyl-1H-imidazo[4, 5-c] quinolin-4-amide hydrochloride (3% suspension, D), 1-butyl-1H-imidazo[4, 5-c] quinolin-4-amide hydrochloride (3% suspension, E), and 1 -benzyl- 1H-imidazo [4, 5-c] quinolin-4-amide hydrochloride (3% suspension, F), crossing isolated human skin tissue in Franz cells (n=5). In each case, the vehicle was a mixture of ethanol/pH 7.4 phosphate buffer (0.2 M) (v/v, 70/30).
Figure 2: Wherein, R represents a branched or straight chain, -(CH₂)ₙ-, wherein n=0, 1, 2, 3, 4, 5, 6, 7, 8, 9, 10, in -(CH₂)ₙ-, any CH₂ may be replaced with O, S, CH=CH, C≡C, CR₁₁R₁₂, aryl or heteroaryl residues; R₁ and R₂ are independently selected from the group consisting of H, one of any alkyl, alkyloxyl, alkenyl, perfluoroalkyl, alkyl halide or alkynyl residues having up to 15 carbon atoms, aryl or heteroaryl moieties or taken together are -(CH₂)ₘ-, wherein m=2, 3, 4, 5, 6, 7, 8, 9, 10, and any CH₂ may be replaced with O, S, CH=CH, C≡C, CR₁₁R₁₂, aryl or heteroaryl moieties; R₃ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C≡C, CR₁₁R₁₂, aryl or heteroaryl moieties; X represents O, NH, or S; Y represents C, S=O, or P-OR₁₁; R₄ is selected from the group consisting of alkyl of 1 to about 10 carbon atoms, hydroxylalkyl of 1 to about 10 carbon atoms, acyloxyalkyl wherein the acyloxy moiety is alkanoyloxy of 2 to about 5 carbon atoms or benzoyloxy, and the alkyl moiety contains 1 to about 8 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to about 5 carbon atoms, alkoxy of 1 to about 5 carbon atoms and halogen, with the proviso that if said benzene ring is substituted by 2 of said moieties, then said moieties together contain no more than 8 carbon atoms; R₅ is selected from the group consisting of hydrogen, alkyl of 1 to about 10 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to about 5 carbon atoms, alkoxy of 1 to about 5 carbon atoms and halogen, with the proviso that if said benzene ring is substituted by 2 of said moieties, then said moieties together contain no more than 8 carbon atoms; R₆ is selected from the group consisting of hydrogen, alkyl of 1 to about 5 carbon atoms, and alkoxy of 1 to about 5 carbon atoms and halogen; R₇ is selected from the group consisting of hydrogen, alkyl of 1 to about 5 carbon atoms, and alkoxy of 1 to about 5 carbon atoms and halogen; R₈ represents a branched or straight chain, -(CH₂)ₐ-, wherein a=0, 1, 2, 3, 4, 5, 6, 7, 8, 9, 10, in -(CH₂)ₐ-, any CH₂ may be replaced with O, S, CH=CH, C≡C, CR₁₁R₁₂, aryl or heteroaryl residues ; R₉ represents a branched or straight chain, -(CH₂)_{b}-, wherein b=0, 1, 2, 3, 4, 5, 6, 7, 8, 9, 10, in -(CH₂)_{b}-, any CH₂ may be replaced with O, S, CH=CH, C≡C, CR₁₁R₁₂, aryl or heteroaryl residues; R₁₀ represents a branched or straight chain,-(CH₂)_{c}-, wherein c=0, 1, 2, 3, 4, 5, 6, 7, 8, 9, 10, in -(CH₂)_{c}-, any CH₂ may be replaced with O, S, CH=CH, C≡C, CR₁₁R₁₂, aryl or heteroaryl residues; R₁₁ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C≡C, CR₁₁R₁₂, aryl or heteroaryl moieties; R₁₂ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C≡C, CR₁₁ R₁₂, aryl or heteroaryl moieties; A⁻ represents a negative ions. All R, R₁, R₂, R₃, R₄, -(CH₂)ₙ-, -(CH₂)ₘ-, -(CH₂)ₐ-, -(CH₂)_{b}-, or -(CH₂)_{c}- groups are branched or straight chains and may include C, H, O, S, P, or N atoms and may have single, double, and triple bonds.

### Best Mode

### Preparation of Boc-sarcosine 1-isobutyl-1H-imidazo[4, 5-c]quinolin-4-amide

32.2 g (0.1 mol) of 1-isobutyl-1H-imidazo[4, 5-c]quinolin-4-amine was dissolved in 500 ml of ethyl acetate. 36 g (0.1 mol) of N-t-Butyloxycarbonyl-N-methylglycine anhydride [(Boc-N-Me-Gly) O] and 20 ml of triethylamine were added into the reaction mixture. The mixture was refluxed for 2 h. The solution was washed with water (1 x 100 ml), 10% citric acid (1 x 100 ml), water (1 x 100 ml), 5% sodium bicarbonate (1 x 100 ml), and water (3 x 100 ml). The ethyl acetate solution is dried over anhydrous sodium sulfate. The ethyl acetate solution was evaporated to dryness. After drying, it yielded 36 g of the desired product (87.5%). Elementary analysis: C₂₂H₂₉N₅O₃; MW: 411.50. Calculated % C: 64.21; H: 7.10; N: 17.02; O: 11.66; Found % C: 64.17; H: 7.15; N: 16.97; O: 11.71. ¹H-NMR (400 MHz, CDCl₃): δ: 1.12 (d, 6H), 1.52 (d, 9H), 2,37 (m, H), 3.05 (s, 3H), 4.30 (d, 2H), 4.78 (m, 2H), 7.60-7.70 (m, 2H), 7.85 (s, H), 8.02-8.20 (m, 2H), 9.01 (b, H).

### Preparation of sarcosine 1-isobutyl-1H-imidazo[4, 5-c]quinolin-4-amide hydrochloride

35 g of Boc-sarcosine 1-isobutyl-1H-imidazo[4, 5-c]quinolin-4-amide was dissolved in ethanol (200 ml). HCl gas was bubbled into the reaction mixture for 20 min. slowly. The mixture was stirred for 1 hour at RT. Ether (500 ml) was added into the mixture. The solid was collected by filtration and washed with ether (3 x). After drying, it yielded 27 g of the desired product (91.2%). Elementary analysis: C17H22C1N5O; MW: 347.84. Calculated % C: 58.70; H: 6.37; Cl: 10.19; N: 20.13; O: 4.60; Found % C: 58.67; H: 6.41; Cl: 10.17; N: 20.11; O: 4.64.

### Mode for Invention

### Preparation of Boc-Glycine 1-butyl-1H-imidazo[4, 5-c]quinolin-4-amide

32.2 g (0.1 mol) of 1-butyl-1H-imidazo[4, 5-c]quinolin-4-amine was dissolved in 300 ml of ethyl acetate. 33.3 g (0.1 mol) of N-t-Butyloxycarbonyl-glycine anhydride [(Boc-Gly)₂O] and 20 ml of triethylamine were added into the reaction mixture. The mixture was refluxed for 2 h. The solution was washed with water (1 x 100 ml), 10% citric acid (1 x 100 ml), water (1 x 100 ml), 5% sodium bicarbonate (1 x 100 ml), and water (3 x 100 ml). The ethyl acetate solution is dried over anhydrous sodium sulfate. The ethyl acetate solution was evaporated to dryness. After drying, it yielded 37 g of the desired product (93.1 %). Elementary analysis: C₂₁ H₂₇ N₅ O₃; MW: 397.47. Calculated % C: 63.46; H: 6.85; N: 17.62; O: 12.08; Found % C: 63.42; H: 6.87; N: 17.60; O: 12.11. ¹H-NMR (400 MHz, CDCl₃): δ: 0.93 (t, 3H), 1.27 (m, 2H), 1.45 (d, 9H), 1.68 (m, 2H), 4.30 (m, 2H), 4.76 (m, 2H), 7.60-7.71 (m, 2H), 7.85 (s, H), 8.02-8.17 (m, 2H), 8.62 (b, H), 9.02 (b, H).

### Preparation of glycine 1-isobutyl-1H-imidazo[4, 5-c]quinolin-4-amide hydrochloride

36 g of Boc-glycine 1-isobutyl-1H-imidazo[4, 5-c]quinolin-4-amide was dissolved in ethanol (200 ml). HCl gas was bubbled into the reaction mixture for 20 min. slowly. The mixture was stirred for 1 hour at RT. Ether (500 ml) was added into the mixture. The solid was collected by filtration and washed with ether (3 x). After drying, it yielded 28 g of the desired product (92.6%). Elementary analysis: C₁₆ H₂₀ CIN₅ O; MW: 333.82. Calculated % C: 57.57; H: 6.04; Cl: 10.62; N: 20.98; O: 4.79; Found % C: 57.52; H: 6.08; Cl: 10.67; N: 20.95; O: 4.78.

### Preparation of N,N-dimethylglycine 1-butyl-1H-imidazo[4,5-c] quinolin-4-amide hydrochloride

Glycine 1-butyl-1H-imidazo[4, 5-c]quinolin-4-amide hydrochloride (27 g) was dissolved in 2N NaOH (50 ml). 50 ml of 40% formaldehyde and 50 ml of acetic acid were added into the reaction mixture. 30 g of NaBH was added into the reaction mixture slowly. After addition, the mixture is stirred for 30 min. Another 25 ml of 40% formaldehyde and 10 ml of acetic acid was added into the reaction mixture. 20 g of NaBH was added into the reaction mixture slowly. The mixture is evaporated to dryness. The residue was purified by silica gel column chromatography. Yielded 20 g of desired product (68.8%). Elementary analysis: C₁₈H₂₄ClN₅O; MW: 361.87. Calculated % C: 59.74; H: 6.68; Cl: 9.80; N: 19.35; O: 4.42; Found % C: 59.72; H: 6.72; Cl: 9.75; N: 19.37; O: 4.44.

### Preparation of Boc-sarcosine 1-benzyl-1H-imidazo[4, 5-c]quinolin-4-amide

18.9 g (0.1 mol) of N-t-Butyloxycarbonyl-N-methylglycine was dissolved in 300 ml of dichloromethlene. 20.6 g of N, N'-Dicyclohexylcarbodiimide was added into the reaction mixture. The mixture was stirred for 1 hour at 0°C. 32.2 g (0.1 mol) of 1-benzyl-1H-imidazo[4, 5-c]quinolin-4-amine and 20 ml of triethylamine were added into the reaction mixture. The mixture was stirred for 3 hours at RT. The solid was removed by filtration. The dichloromethlene solution was washed with water (1 x 100 ml), 30% citric acid (1 x 100 ml), water (1 x 100 ml), 5% NaHCO (2 x 100 ml), and water (3 x 100 ml). The organic solution was dried over anhydrous sodium sulfate. Sodium sulfate was removed by filtration. The organic solution was evaporated to dryness. After drying, it yielded 33 g of the desired product (74.1 %). Elementary analysis: C₂₅H₂₇N₅O₃; MW: 445.51. Calculated % C: 67.40; H: 6.11; N: 15.72; O: 10.77; Found % C: 67.35; H: 6.14; N: 15.70; O: 10.81. ¹H-NMR (400 MHz, CDCl₃): δ: 1.52 (d, 9H), 3.02 (s, 3H), 4.68 (m, 2H), 4.98 (m, 2H), 7.10-7.16 (m, 5H), 7.60-7.70 (m, 2H), 7.85 (s, H), 8.02-8.20 (m, 2H), 8.91 (b, H).

### Preparation of sarcosine 1-benzyl-1H-imidazo[4, 5-c]quinolin-4-amide hydrochloride

32 g of Boc-sarcosine 1-benzyl-1H-imidazo[4, 5-c]quinolin-4-amide was dissolved in ethanol (200 ml). HCl gas was bubbled into the reaction mixture for 20 min. slowly. The mixture was stirred for 1 hour at RT. Ether (500 ml) was added into the mixture. The solid was collected by filtration and washed with ether (3 x). After drying, it yielded 25 g of the desired product (85.7%). Elementary analysis: C₂₀H₂₀ClN₅O; MW: 381.86. Calculated % C: 62.91; H: 5.28; Cl: 9.28; N: 18.34; O: 4.19; Found % C: 62.87; H: 5.31; Cl: 9.30; N: 18.32; O: 4.20.

### Industrial Applicability

The pro-drugs of the general formula (1) 'Structure 1' or formula (2) 'Structure 2' are superior to 1H-imidazo[4, 5-c]quinolin-4-amines and related compounds. They can be used medicinally in treating any 1H-imidazo[4, 5-c]quinolin-4-amines and related compounds-treatable conditions in humans or animals. They may be used for the treatment of actinic keratosis, basal cell carcinoma, cancers, AIDs, bird flu, genital and perianal warts, and other virus diseases.

## Claims

1. A compound having a general formula of Structure 1 or Structure 2, 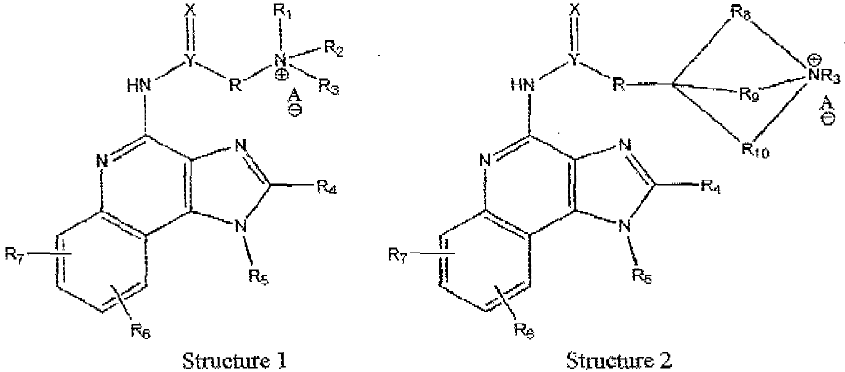 wherein,
R represents a branched or straight chain, -(CH₂)ₙ-, wherein n= 0, 1, 2, 3, 4, 5, 6, 7, 8, 9, 10, in -(CH₂)ₙ-, any CH₂ may be replaced with O, S, CH=CH, C≡C, or CR₁₁R₁₂;
R₁ and R₂ are independently selected from the group consisting of H, alkyl having 1 to 15 carbon atoms, alkyloxy, having 1 to 15 carbon atoms, alkenyl having up to 15 carbon atoms, perfluoroalkyl having 1 to 15 carbon atoms, alkyl halide having 1 to 15 carbon atoms, alkynyl having up to 15 carbon atoms, aryl, and heteroaryl moieties, or taken together to -(CH₂)ₘ-, wherein m= 2, 3, 4, 5, 6, 7, 8, 9, 10, and any CH₂ may be replaced with O, S, CH=CH, C≡C ;
R₃ is selected from the group consisting of H, alkyl having 1 to 15 carbon atoms, alkyloxy, having 1 to 15 carbon atoms, alkenyl having up to 15 carbon atoms, perfluoroalkyl having 1 to 15 carbon atoms, alkyl halide having 1 to 15 carbon atoms, alkynyl having up to 15 carbon atoms, aryl and heteroaryl moieties, wherein, any CH₂ may be replaced with O, S, CH=CH, C≡C, or CR₁₁R₁₂;
X represents O, NH, or S;
Y represents C, S=O, or P-OR₁₁;
R₄ is selected from the group consisting of alkyl of 1 to 10 carbon atoms, hydroxylalkyl of 1 to 10 carbon atoms, acyloxyalkyl wherein the acyloxy moiety is alkanoyloxy of 2 to 5 carbon atoms or benzoyloxy, and the alkyl moiety contains 1 to 8 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to 5 carbon atoms, alkoxy of 1 to 5 carbon atoms and halogen, with the proviso that if said benzene ring is substituted by 2 of said moieties, then said moieties together contain no more than 8 carbon atoms;
R₅ is selected from the group consisting of hydrogen, alkyl of 1 to 10 carbon atoms, benzyl, (phenyl)ethyl and phenyl, said benzyl, (phenyl)ethyl or phenyl substituent being optionally substituted on the benzene ring by 1 or 2 moieties independently selected from the group consisting of alkyl of 1 to 5 carbon atoms, alkoxy of 1 to 5 carbon atoms and halogen, with the proviso that if said benzene ring is substituted by 2 of said moieties, then said moieties together contain no more than 8 carbon atoms;
R₆ is selected from the group consisting of hydrogen, alkyl of 1 to 5 carbon atoms, and alkoxy of 1 to 5 carbon atoms and halogen;
R₇ is selected from the group consisting of hydrogen, alkyl of 1 to 5 carbon atoms, and alkoxy of 1 to 5 carbon atoms and halogen;
R₈ represents a branched or straight chain, -(CH₂)ₙ-, wherein a= 0, 1, 2, 3, 4, 5, 6, 7, 8, 9, 10, in -(CH₂)ₙ-, any CH₂ may be replaced with O, S, CH=CH, C≡C, or CR₁₁R₁₂;
R₉ represents a branched or straight chain, -(CH₂)_{b}-, wherein b= 0, 1, 2, 3, 4, 5, 6, 7, 8, 9, 10, in -(CH₂)_{b}-, any CH₂ may be replaced with O, S, CH=CH, C≡C, or CR₁₁R₁₂;
R₁₀ represents a branched or straight chain, -(CH₂)_{c}-, wherein c= 0, 1, 2, 3, 4, 5, 6, 7, 8, 9, 10, in -(CH₂)_{c}-, any CH₂ may be replaced with O, S, CH=CH, C=C, or CR₁₁R₁₂;
R₁₁ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C≡C, or CR₁R₂;
R₁₂ represents H, one of any alkyl, alkyloxy, alkenyl, perfluoroalkyl, alkyl halide, or alkynyl residues having up to 15 carbon atoms, aryl or heteroaryl moieties, wherein, any CH₂ maybe replaced with O, S, CH=CH, C≡C, or CR₁R₂;
A⁻ represents a negative ion;
and all R, R₁, R₂, R₃, R₄, , -(CH₂)ₙ-, -(CH₂)ₘ-, -(CH₂)ₐ-, -(CH₂)_{b}-, or -(CH₂)_{c}-groups are branched or straight chains and may include C, H, O, S, P, or N atoms and may have single, double, and triple bonds.

2. A compound selected from the group consisting of sarcosine 1-isobutyl-1H-imidazo[4, 5-c] quinolin-4-amide.HA, N,N-dimethylglycine 1-isobutyl-1H-imidazo[4, 5-c]quinolin-4-aroide.HA, sarcosine 1-butyl-1H-imidazo[4, 5-c]quinolin-4-amide.HA, sarcosine 1-benzyl-1H-imidazo[4, 5-c]quxnolin-4-amide.HA, sarcosine 1-methyl-H-imidazo [4, 5-c] quinolin-4-amide.HA, sarcosine 1, 2, 8-trimethyl-1H-imidazo[4, 5-c]quinolin-4-amide.HA, sarcosine 1-(2-hydroxyethyl)-1H-imidazo[4,5-c]quinolin-4-amide.HA, sarcosine 1, 8-dimethyl-1H-imidazo[4, 5-c]quinolin-4-amide.HA, sarcosine 1, 2-dimethyl-1H-imidazo[4, 5-c]quinolin-4-amide.HA, sarcosine 1-(2,3-dihydroxypropyl)-1H-imidazo[4, 5-c]quinolin-4-amide.HA, sarcosine 1-cyclohexylmethyl-1H-imidazo[4, 5-c]quinolin-4-amide.HA, sarcosine 1-benzyl-2-methyl-1H-imidazo[4,5-c]quinolin-4-amide.HA, sarcosine 1-n-hexyt-1H-imidazo[4, 5-c]quinolin-4-amide.HA, and sarcosine 1-n-hexyl-2-methyl-1H-imidazo[4,5-c]quinolin-4-amide.HA, wherein, A⁻ represents a negative ion.

3. Processes for the preparation of a compound having a general formula of Structure 1 or Structure 2 according to claim 1 or a compound according to claim 2, wherein the compound is prepared from 1H-imidazo[4, 5-c]quinolin-4-amines or related compounds, by reaction with a compound having a general formula of Structure 3 using a coupling reagent selected from the group consisting of N,N'-dicyclohexylcarbodiimide, N,N'-diisopropylcarbodiimide, O-(benzotriazol-1-yl)-N,N,N',N'-tetramethyluronium tetrafluoroborate, O-(benzotriazol-1-yl)-N,N,N',N'-tetramethyluronium hexafluorophosphate, and benzotriazol-1-yl-oxy-tris(dimethylamino)phosphonium hexafluorophosphate, 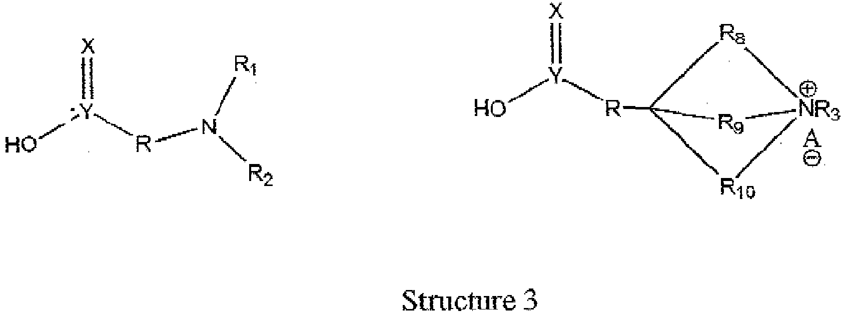
wherein, R represents a branched or straight chain, -(CH₂)ₙ-, wherein n= 0, 1, 2, 3, 4, 5, 6, 7, 8, 9, 10, in -(CH₂)ₙ-, any CH₂ may be replaced with O, S, CH=CH, C≡C, CR₁₁R₁₂, aryl or heteroaryl residues;
R₁ and R₂ taken alone are same or different and are H, one of any alkyl, alkyloxy, alkenyl or alkynyl residues having up to 15 carbon atoms, aryl or heteroaryl moieties or taken together are -(CH₂)ₘ-, wherein m= 2, 3, 4, 5, 6, 7, 8, 9, 10, and any CH may be replaced with O, S, CH=CH, C≡C, aryl or heteroaryl moieties;
X represents O, NH, or S;
Y represents C, S=O, or P-OR₁₁;
R₃ represents H, one of any alkyl, Alkyloxy, alkenyl or alkynyl residues having up to 15 carbon atoms, aryl or heteroaryl moieties;
R₈ represents a branched or straight chain, -(CH₂)ₐ-, wherein a= 0, 1, 2, 3. 4, 5, 6, 7, 8, 9, 10, in -(CH₂)ₐ-, any CH₂ may be replaced with O, S, CH=CH, C≡C, CR₁R₂, aryl or heteroaryl residues;
R₉ represents a branched or straight chain, -(CH₂)_{b}-, wherein b= 0, 1, 2, 3, 4, 5, 6, 7, 8, 9, 10, in -(CH₂)_{b}-, any CH₂ may be replaced with O, S, CH=CH, C≡C, CR₁₁R₁₂, aryl or heteroaryl residues;
R₁₀ represents a branched or straight chain, -(CH₂)_{c}-, wherein c= 0, 1, 2, 3, 4, 5, 6, 7, 8, 9, 10, in -(CH₂)_{c}-, any CH₂ may be replaced with O, S, CH=CH, C≡C, CR₁₁R₁₂, aryl or heteroaryl residues;
R₁₁ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C≡C, CR ₁R ₂, aryl or heteroaryl moieties;
R₁₂ represents H, one of any alkyl, alkyloxy, alkenyl, perfluoroalkyl, alkyl halide, or alkynyl residues having up to 15 carbon atoms, aryl or heteroaryl moieties, wherein, any CH₂ may be replaced with O, S, CH=CH, C≡C, CR₁R₂-, aryl or heteroaryl, moieties;
A⁻ represents a negative ion;
and all R, R₁, R₂, R₃, R₄, -(CH₂)ₙ-, -(CH₂)ₘ-, -(CH₂)ₐ-, -(CH₂)_{b}-, or -(CH₂)_{c}- groups are branched or straight chains and may include C, H, O, S, P, or N atoms and may have single, double, and triple bonds.

4. A compound as in any one of claims 1 to 3, wherein A represents Cl⁻, Br⁻, F⁻, I⁻, AcO⁻ or citrate.

5. The compound according to claim 1 or claim 2, or a composition comprising at least one compound according to claim 1 or claim 2, for use in the treatment of treating a 1H-imidazo[4,5-c]quinolin-4-amines or related compounds-treatable condition in humans or animals, wherein the 1H-imidazo[4, 5-c]quinolin-4-amines or related compounds-treatable condition is selected from the group consisting of acquired immune deficiency syndrome (AIDS), bird flu, hydrophobia (rabies infection), warts, severe acute respiratory syndrome (SARS), eczema, hepatitis, psoriasis, multiple sclerosis, essential thrombocythaemia, virus diseases, actinic keratosis, basal cell carcinoma, skin cancers, breast cancer, colon cancer, lung cancer, oral cancers and cancers; and the compound or the composition is administered orally or transdermally.

6. The compound or composition for use according to claim 5, wherein the compound or composition is in the form of a solution, spray, lotion, ointment, emulsion or gel, and the compound or composition is administered transdermally.

7. The compound according to claim 1 or claim 2 or a composition comprising at least one compound according to claim 1 or claim 2 as an active ingredient for use in the treatment or the prevention of acquired immune deficiency syndrome (AIDS), wherein the compound or composition is in the form of a solution, spray, lotion, ointment, emulsion, or gel, wherein the compound according to claim 1 or claim 2 is employed at therapeutically effective levels and the compound or composition is administered transdermally to the upper respiratory tract and/or the genital area of a male or female human.

8. A condom for use in the prevention of acquired immune deficiency syndrome (AIDS), wherein the condom comprises
(1) condom materials mixed with a compound according to claim 1 or claim 2 or a composition comprising at least one compound according to claim 1 or claim 2, or
(2) a coating comprising a compound according to claim 1 or claim 2 or a composition comprising at least one compound according to claim 1 or claim 2,
wherein the compound or composition acts as an active ingredient, therapeutically effective levels of a compound according to claim 1 or claim 2 are employed, and the coating is in the form of a solution, spray, lotion, ointment, emulsion, or gel.

9. Transdermal therapeutic application systems of a compound according to claim 1 or claim 2 for use in the treatment of acquired immune deficiency syndrome (AIDS), bird flu, hydrophobia (rabies infection), warts, severe acute respiratory syndrome (SARS), eczema, hepatitis, psoriasis, multiple sclerosis, essential thrombocythaemia, virus diseases, actinic keratosis, basal cell carcinoma, skin cancers, breast cancer, colon cancer, lung cancer, oral cancers and cancers.

10. Transdermal therapeutic application systems according to claim 9, wherein the systems comprises a bandage or a patch comprising of one active substance-containing matrix layer and an impermeable backing layer.

11. Transdermal therapeutic application systems according to claim 9, wherein the system comprises an active substance reservoir, which has a permeable bottom facing the skin, wherein controlling the rate of release, this system enables the 1H-imidazo[4, 5-c] quinolin-4-amines and related compounds to reach constantly optimal therapeutic blood levels to increase effectiveness and reduce the side effects of 1H-imidazo[4, 5-c]quinolin-4-amines and related compounds.

12. The compound according to claim 1 or claim 2, or a composition comprising at least one compound according to claim 1 or claim 2 for use in the treatment of cancer, wherein the compound or composition is further combined with a second composition, the second composition is selected from the group consisting of nonsteroidal anti-inflammatory drugs, prodrugs thereof, diethylaminoethyl 2-(pisobutylphenyl) propionate.AcOH, diethylaminoethyl 1-(pehlorobenzoyl)-5-methoxy-2-methylindole 3-acetate.AcOH, diethylaminoethyl (Z)-5-fluoro-2-methyl-1-[(4-methylsulfinyl) phenylmethylene]-1H-indene-3-acetate.AcOH, diethylaminoethyl 1-methyl-5-(4-methylbenzoyl)-1H-pyrrole-2-acetate.AcOH, diethylaminoethyl 5-(4-Chlorobemoyl)-1,4-dimethyl-1Hpyrrole-2-acetate.AcOH, and diethylaminoethyl 1,9-diethyl-1,3,4,9-tetrahydropyrano-[3,4-b]indole-1-acetate.AcOH.

13. The compound according to claim 1 or claim 2, or a composition comprising at least one compound according to claim 1 or claim 2 as an active ingredient for use in the treatment of cancer, wherein the compound or composition is administered orally or transdermally before or/and after removing tumor surgery.

## Patentansprüche

1. Verbindung mit einer allgemeinen Formel der Struktur 1 oder Struktur 2, 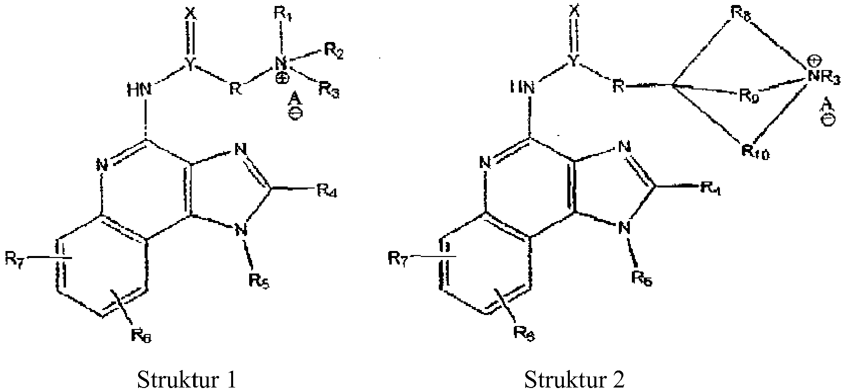 wobei
R eine verzweigte oder gerade Kette repräsentiert, -(CH₂)ₙ-, wobei n= 0, 1, 2, 3, 4, 5, 6, 7, 8, 9, 10, in -(CH₂)ₙ-jedes CH₂ durch O, S, CH=CH, C≡C oder CR₁₁R₁₂ ersetzt werden kann;
R₁ und R₂ unabhängig aus der Gruppe ausgewählt sind, bestehend aus H, Alkyl mit 1 bis 15 Kohlenstoffatomen, Alkyloxy mit 1 bis 15 Kohlenstoffatomen, Alkenyl mit bis zu 15 Kohlenstoffatomen, Perfluoralkyl mit 1 bis 15 Kohlenstoffatomen, Alkylhalid mit 1 bis 15 Kohlenstoffatomen, Alkynyl mit bis zu 15 Kohlenstoffatomen, Aryl- und Heteroarylteilen, oder zusammengefasst zu -(CH₂)ₘ-, wobei m= 2, 3, 4, 5, 6, 7, 8, 9, 10, und jedes CH₂ durch O, S, CH=CH, C≡C ersetzt werden kann;
R₃ aus der Gruppe ausgewählt ist, bestehend aus H, Alkyl mit 1 bis 15 Kohlenstoffatomen, Alkyloxy mit 1 bis 15 Kohlenstoffatomen, Alkenyl mit bis zu 15 Kohlenstoffatomen, Perfluoralkyl mit 1 bis 15 Kohlenstoffatomen, Alkylhalid mit 1 bis 15 Kohlenstoffatomen, Alkynyl mit bis zu 15 Kohlenstoffatomen, Aryl- und Heteroarylteilen, wobei jedes CH₂ durch O, S, CH=CH, C≡C oder CR₁₁R₁₂ ersetzt werden kann;
X O, NH oder S repräsentiert;
Y C, S=O oder P-OR₁₁ repräsentiert;
R₄ aus der Gruppe ausgewählt ist, bestehend aus Alkyl mit 1 bis 10 Kohlenstoffatomen, Hydroxyalkyl mit 1 bis 10 Kohlenstoffatomen, Acyloxyalkyl, wobei der Acyloxyteil Alkanoyloxy mit 2 bis 5 Kohlenstoffatomen oder Benzoyloxy ist, und der Alkylteil 1 bis 8 Kohlenstoffe enthält, Benzyl, (Phenyl)ethyl und Phenyl, wobei der Benzyl-, (Phenyl)ethyl- oder Phenylsubstituent auf dem Benzolring optional durch 1 oder 2 Teile substituiert wird, die unabhängig aus der Gruppe ausgewählt sind, bestehend aus Alkyl mit 1 bis 5 Kohlenstoffen, Alkoxy mit 1 bis 5 Kohlenstoffen und Halogen, mit der Maßgabe, dass, wenn der Benzolring durch 2 der Teile substituiert wird, die Teile dann zusammen nicht mehr als 8 Kohlenstoffatome enthalten;
R₅ aus der Gruppe ausgewählt ist, bestehend aus Wasserstoff, Alkyl mit 1 bis 10 Kohlenstoffatomen, Benzyl, (Phenyl)ethyl und Phenyl, wobei der Benzyl-, (Phenyl)ethyl- oder Phenylsubstituent auf dem Benzolring optional durch 1 oder 2 Teile substituiert wird, die unabhängig aus der Gruppe ausgewählt sind, bestehend aus Alkyl mit 1 bis 5 Kohlenstoffen, Alkoxy mit 1 bis 5 Kohlenstoffen und Halogen, mit der Maßgabe, dass, wenn der Benzolring durch 2 der Teile substituiert wird, die Teile dann zusammen nicht mehr als 8 Kohlenstoffatome enthalten;
R₆ aus der Gruppe ausgewählt ist, bestehend aus Wasserstoff, Alkyl mit 1 bis 5 Kohlenstoffatomen und Alkoxy mit 1 bis 5 Kohlenstoffatomen und Halogen;
R₇ aus der Gruppe ausgewählt ist, bestehend aus Wasserstoff, Alkyl mit 1 bis 5 Kohlenstoffatomen und Alkoxy mit 1 bis 5 Kohlenstoffatomen und Halogen;
R₈ eine verzweigte oder gerade Kette repräsentiert, -(CH₂)ₙ-, wobei a= 0, 1, 2, 3, 4, 5, 6, 7, 8, 9, 10, in -(CH₂)ₙ jedes CH₂ durch O, S, CH=CH, C≡C oder CR₁₁R₁₂ ersetzt werden kann;
R₉ eine verzweigte oder gerade Kette repräsentiert, -(CH₂)_{b}-, wobei b= 0, 1, 2, 3, 4, 5, 6, 7, 8, 9, 10, in -(CH₂)_{b}- jedes CH₂ durch O, S, CH=CH, C≡C oder CR₁₁R₁₂ ersetzt werden kann;
R₁₀ eine verzweigte oder gerade Kette repräsentiert, -(CH₂)_{c}-, wobei c- 0, 1, 2, 3, 4, 5, 6, 7, 8, 9, 10, in -(CH₂)_{c}-jedes CH₂ durch O, S, CH=CH, C≡C oder CR₁₁R₁₂ ersetzt werden kann;
R₁₁ H, eines von einem Alkyl, Alkyloxy, Alkenyl, Perfluoralkyl, Alkylhalid oder Alkynylrückstände mit bis zu 15 Kohlenstoffatomen, Aryl- oder Heteroarylteilen repräsentiert, wobei jedes CH₂ durch O, S, CH=CH, C≡C oder CR₁R₂ ersetzt werden kann;
R₁₂ H, eines von einem Alkyl, Alkyloxy, Alkenyl, Perfluoralkyl, Alkylhalid oder Alkynylrückstände mit bis zu 15 Kohlenstoffatomen, Aryl- oder Heteroarylteilen repräsentiert, wobei jedes CH₂ durch O, S, CH=CH, C≡C oder CR₁R₂ ersetzt werden kann;
A⁻ ein negatives Ion repräsentiert;
und alle R-, R₁-, R₂-, R₃-, R₄-, -(CH₂)ₙ-, -(CH₂)ₘ-, -(CH₂)ₐ-, -(CH₂)_{b}- oder (CH₂)_{c}-Gruppen verzweigte oder gerade Ketten sind und C-, H-, O-, S-, P- oder N-Atome enthalten können und Einfach-, Doppel- und Dreifachbindungen aufweisen können.

2. Verbindung ausgewählt aus der Gruppe, bestehend aus Sarcosin-1-isobutyl-1H-imidazo[4,5-c]quinolin-4-amid.HA, N,N-Dimethylglycin-1-isobutyl-1H-imidazo[4,5-c]quinolin-4-amid.HA, Sarcosin-1-butyl-1H-imidazo[4,5-c]quinolin-4-amid.HA, Sarcosin-1-benzyl-1H-imidazo[4,5-c]quinolin-4-amid.HA, Sarcosin-1-methyl-H-imidazo[4,5-c]quinolin-4-amid.HA, Sarcosin-1,2,8-trimethyl-1H-imidazo[4,5-c]quinolin-4-amid,HA, Sarcosin-1(2-hydroxyethyl)-1H-imidazo[4,5-c]quinolin-4-amid.HA, Sarcosin-1,8-dimethyl-1H-imidazo[4,5-c]quinolin-4-amid.HA, Sarcosin-1,2-dimethyl-1H-imidazo[4,5-c]quinolin-4-amid.HA, Sarcosin-1(2,3-dihydroxypropyl)-1H-imidazo[4,5-c]quinolin-4-amid.HA, Sarcosin-1-cyclohexylmethyl-1H-imidazo[4,5-c]quinolin-4-amid.HA, Sarcosin-1-benzyl-2-methyl-1H-imidazo[4,5-c]quinolin-4-amid.HA, Sarcosin-1-n-hexyl-1H-imidazo[4,5-c]quinolin-4-amid.HA und Sarcosin-1-n-hexyl-2-methyl-1H-imidazo[4,5-c]quinolin-4-amid.HA, wobei A⁻ ein negatives Ion repräsentiert.

3. Verfahren für die Herstellung einer Verbindung mit einer allgemeinen Formel der Struktur 1 oder Struktur 2 gemäß Anspruch 1 oder einer Verbindung gemäß Anspruch 2, wobei die Verbindung hergestellt ist aus 1H-Imidazo[4,5-c]quinolin-4-aminen oder verwandten Verbindungen; durch Reaktion mit einer Verbindung mit einer allgemeinen Formel der Struktur 3 unter Verwendung eines Kopplungsreagens, ausgewählt aus der Gruppe bestehend aus N,N'-Dicyclohexylcarbodiimid, N,N'-Diisopropylcarbodiimid, O-(Benzotriazol-1-yl)-N,N,N',N'-tetramethyluroniumtetrafluorborat, O-(Benzotriazol-1-yl)-N,N,N',N'-tetramethyluroniumhexafluorphosphat und Benzotriazol-1-yl-oxy-tris(dimethylamino)phosphoniumhexafluorphosphat, 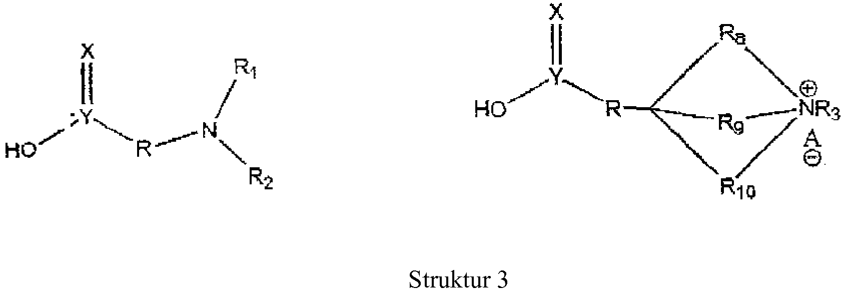
wobei R eine verzweigte oder gerade Kette repräsentiert, -(CH₂)ₙ-, wobei n= 0, 1, 2, 3, 4, 5, 6, 7, 8, 9, 10, in -(CH₂)ₙ- jedes CH₂ durch O, S, CH=CH, C≡C, CR₁₁R₁₂, Aryl- oder Heteroarylrückstände ersetzt werden kann;
R₁ und R₂ alleine genommen gleich oder unterschiedlich sind und H, eines von einem Alkyl-, Alkyloxy-, Alkenyl- oder Alkynylrückstand mit bis zu 15 Kohlenstoffatomen, Aryl- oder Heteroarylteile oder zusammengenommen -(CH₂)ₘ- sind, wobei m= 2, 3, 4, 5, 6, 7, 8, 9, 10 und jedes CH durch O, S, CH=CH, C≡C, Aryl- oder Heteroarylteile ersetzt werden kann;
X O, NH oder S repräsentiert;
Y C, S=O oder P-OR₁₁ repräsentiert;
R₃ H, eines von einem Alkyl-, Alkyloxy-, Alkenyl- oder Alkynylrückstand mit bis zu 15 Kohlenstoffatomen, Aryl- oder Heteroarylteile repräsentiert;
R₈ eine verzweigte oder gerade Kette repräsentiert, -(CH₂)ₐ, wobei a= 0, 1, 2, 3, 4, 5, 6, 7, 8, 9, 10, in -(CH₂)ₐ-jedes CH₂ durch O, S, CH=CH, C≡C, CR₁R₂, Aryl- oder Heteroarylrückstände ersetzt werden kann;
R₉ eine verzweigte oder gerade Kette repräsentiert, -(CH₂)_{b}-, wobei b= 0, 1, 2, 3, 4, 5, 6, 7, 8, 9, 10, in -(CH₂)_{b}-, jedes CH₂ durch O, S, CH=CH, C≡C, CR₁₁R₁₂, Aryl- oder Heteroarylrückstände ersetzt werden kann;
R₁₀ eine verzweigte oder gerade Kette repräsentiert, -(CH₂)_{c}-, wobei c= 0, 1, 2, 3, 4, 5, 6, 7, 8, 9, 10, in -(CH₂)_{c}- jedes CH₂ durch O, S, CH=CH, C≡C, CR₁₁R₁₂, Aryl- oder Heteroarylrückstände ersetzt werden kann;
R₁₁ H, eines von einem Alkyl-, Alkyloxy-, Alkenyl-, Perfluoralkyl-, Alkylhalid- oder Alkynylrückstand mit bis zu 15 Kohlenstoffatomen, Aryl- oder Heteroarylteilen repräsentiert, wobei jedes CH₂ durch O, S, CH=CH, C≡C, CR₁R₂, Aryl- oder Heteroarylteile ersetzt werden kann;
R₁₂ H, eines von einem Alkyl-, Alkyloxy-, Alkenyl-, Perfluoralkyl-, Alkylhalid- oder Alkynylrückstand mit bis zu 15 Kohlenstoffatomen, Aryl- oder Heteroarylteilen repräsentiert, wobei jedes CH₂ durch O, S, CH=CH, C≡C, CR₁R₂-, Aryl- oder Heteroarylteile ersetzt werden kann;
A⁻ ein negatives Ion repräsentiert;
und alle R-, R₁-, R₂-, R₃-, R₄-, -(CH₂)ₙ-, -(CH₂)ₘ-, -(CH₂)ₐ-, -(CH₂)_{b}- oder -(CH₂)_{c}-Gruppen verzweigte oder gerade Ketten sind und C-, H-, O-, S-, P- oder N-Atome enthalten können und Einfach-, Doppel- und Dreifachbindungen aufweisen können.

4. Verbindung nach einem der Ansprüche 1 bis 3, wobei A Cl⁻, Br⁻, F⁻, I⁻, AcO⁻ oder Citrat repräsentiert.

5. Verbindung nach Anspruch 1 oder Anspruch 2, oder Zusammensetzung, umfassend mindestens eine Verbindung nach Anspruch 1 oder Anspruch 2, zur Verwendung in der Behandlung beim Behandeln einer mit 1H-Imidazo[4,5-c]quinolin-4-aminen oder verwandten Verbindungen behandelbaren Erkrankung bei Menschen oder Tieren, wobei die mit 1H-Imidazo[4,5-c]quinolin-4-aminen oder verwandten Verbindungen behandelbare Erkrankung aus der Gruppe ausgewählt ist, bestehend aus erworbenem Immunschwächesyndrom (AIDS), Vogelgrippe, Hydrophobie (Tollwutinfektion), Warzen, schwerem akuten respiratorischen Syndrom (SARS), Ekzemen, Hepatitis, Psoriasis, multipler Sklerose, essentieller Thrombozythämie, Viruserkrankungen, aktinischer Keratose, Basalzellkarzinom, Hautkrebsen, Brustkrebs, Darmkrebs, Lungenkrebs, oralen Krebsen und Krebsen; und die Verbindung oder die Zusammensetzung wird oral oder transdermal verabreicht.

6. Verbindung oder Zusammensetzung zur Verwendung gemäß Anspruch 5, wobei die Verbindung oder Zusammensetzung in der Form einer Lösung, eines Sprays, einer Lotion, einer Salbe, einer Emulsion oder eines Gels ist, und die Verbindung oder Zusammensetzung transdermal verabreicht wird.

7. Verbindung gemäß Anspruch 1 oder Anspruch 2 oder Zusammensetzung, umfassend mindestens eine Verbindung gemäß Anspruch 1 oder Anspruch 2 als aktiven Inhaltsstoff zur Verwendung bei der Behandlung oder der Prävention des erworbenen Immunschwächesyndroms (AIDS), wobei die Verbindung oder Zusammensetzung in der Form einer Lösung, eines Sprays, einer Lotion, einer Salbe, einer Emulsion oder eines Gels ist, wobei die Verbindung gemäß Anspruch 1 oder Anspruch 2 in therapeutisch wirksamen Mengen eingesetzt wird und die Verbindung oder Zusammensetzung transdermal in den oberen Atemweg und/oder den Genitalbereich eines männlichen oder weiblichen Menschen verabreicht wird.

8. Kondom zur Verwendung bei der Prävention des erworbenen Immunschwächesyndroms (AIDS), wobei das Kondom Folgendes umfasst:
(1) Kondommaterialien vermischt mit einer Verbindung gemäß Anspruch 1 oder Anspruch 2 oder einer Zusammensetzung, umfassend mindestens eine Verbindung gemäß Anspruch 1 oder Anspruch 2, oder
(2) eine Beschichtung, umfassend eine Verbindung gemäß Anspruch 1 oder Anspruch 2 oder eine Zusammensetzung, umfassend mindestens eine Verbindung gemäß Anspruch 1 oder Anspruch 2,
wobei die Verbindung oder Zusammensetzung als aktiver Inhaltsstoff agiert, therapeutisch wirksame Mengen einer Verbindung gemäß Anspruch 1 oder Anspruch 2 eingesetzt werden, und die Beschichtung in der Form einer Lösung, eines Sprays, einer Lotion, einer Salbe, einer Emulsion oder eines Gels ist.

9. Transdermale therapeutische Anwendungssysteme einer Verbindung gemäß Anspruch 1 oder Anspruch 2 zur Verwendung bei der Behandlung des erworbenen Immunschwächesyndroms (AIDS), von Vogelgrippe, Hydrophobie (Tollwutinfektion), Warzen, des schweren akuten respiratorischen Syndroms (SARS), von Ekzemen, Hepatitis, Psoriasis, multipler Sklerose, essentieller Thrombozythämie, Viruserkrankungen, aktinischer Keratose, Basalzellkarzinom, Hautkrebsen, Brustkrebs, Darmkrebs, Lungenkrebs, oralen Krebsen und Krebsen.

10. Transdermale therapeutische Anwendungssysteme nach Anspruch 9, wobei die Systeme eine Bandage oder ein Pflaster umfassen, umfassend eine aktive Substanz enthaltende Matrixschicht und eine undurchlässige Stützschicht.

11. Transdermale therapeutische Anwendungssysteme nach Anspruch 9, wobei das System einen Behälter mit einer aktiven Substanz umfasst, der einen durchlässigen Boden aufweist, der der Haut zugewandt ist, wobei durch das Steuern der Freisetzungsrate dieses System den 1H-Imidazo[4,5-c]quinolin-4-aminen und verwandten Verbindungen ermöglicht, konstant optimale therapeutische Blutwerte zu erreichen, um die Wirksamkeit zu erhöhen und die Nebenwirkungen von 1H-Imidazo[4,5-c]quinolin-4-aminen und verwandten Verbindungen zu reduzieren.

12. Verbindung nach Anspruch 1 oder Anspruch 2, oder Zusammensetzung, umfassend mindestens eine Verbindung nach Anspruch 1 oder Anspruch 2 zur Verwendung bei der Behandlung von Krebs, wobei die Verbindung oder Zusammensetzung weiter mit einer zweiten Zusammensetzung kombiniert wird, wobei die zweite Zusammensetzung aus der Gruppe ausgewählt ist, bestehend aus nichtsteroidalen entzündungshemmenden Arzneimitteln, Prodrugs davon, Diethylaminoethyl 2-(pisobutylphenyl)propionat.AcOH, Diethylaminoethyl 1-(pchlorbenzoyl)-5-methoxy-2-methylindol 3-acetat.AcOH, Diethylaminoethyl (Z)-5-fluor-2-methyl-1-[(4-methylsulfinyl) phenylmethylen]-1H-inden-3-acetat.AcOH, Diethylaminoethyl 1-methyl-5-(4- methylbenzoyl)-1H-pyrrol-2-acetat.AcOH, Diethylaminoethyl 5-(4-Chlorbenzoyl)-1,4- dimethyl-lHpyrrol-2-acetat.AcOH und Diethylaminoethyl 1,8-diethyl-1,3,4,9-tetrahydropyrano-[3,4-b]indol-1-acetat.AcOH.

13. Verbindung nach Anspruch 1 oder Anspruch 2, oder Zusammensetzung, umfassend mindestens eine Verbindung nach Anspruch 1 oder Anspruch 2 als aktiven Inhaltsstoff zur Verwendung bei der Behandlung von Krebs, wobei die Verbindung oder Zusammensetzung vor oder/und nach einem chirurgischen Eingriff zur Tumorentfernung oral oder transdermal verabreicht wird.

## Revendications

1. Composé ayant une formule générale selon la Structure 1 ou la Structure 2, 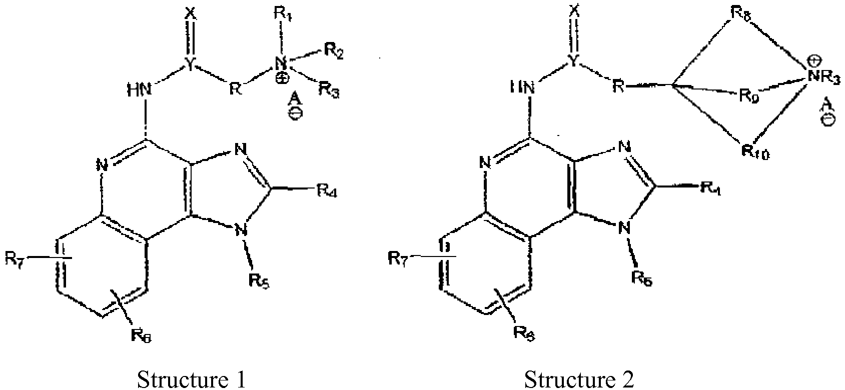 dans lequel,
R représente une chaîne ramifiée ou droite, -(CH₂)ₙ-, dans laquelle n= 0, 1, 2, 3, 4, 5, 6, 7, 8, 9, 10, dans -(CH₂)ₙ-, tout CH₂ peut être remplacé par O, S, CH=CH, C≡C, ou CR₁₁R₁₂,
R₁ et R₂ sont choisis indépendamment dans le groupe comprenant H, un groupe alkyle ayant 1 à 15 atomes de carbone, un groupe alkyloxy ayant 1 à 15 atomes de carbone, un groupe alcényle ayant jusqu'à 15 atomes de carbone, un groupe perfluoroalkyle ayant 1 à 15 atomes de carbone, un halogénure d'alkyle ayant 1 à 15 atomes de carbone, un groupe alkynyle ayant jusqu'à 15 atomes de carbone, un group aryle, et des groupements hétéroaryle, ou pris ensemble à -(CH₂)ₘ-, où m= 2, 3, 4, 5, 6, 7, 8, 9, 10, et tout CH₂ peut être remplacé par O, S, CH=CH, C≡C ;
R₃ est choisi dans le groupe comprenant H, un groupe alkyle ayant 1 à 15 atomes de carbone, un groupe alkyloxy ayant 1 à 15 atomes de carbone, un groupe alcényle ayant jusqu'à 15 atomes de carbone, un groupe perfluoroalkyle ayant 1 à 15 atomes de carbone, un halogénure d'alkyle ayant 1 à 15 atomes de carbone, un groupe alkynyle ayant jusqu'à 15 atomes de carbone, des groupements aryle et hétéroaryle, où tout CH₂ peut être remplacé par O, S, CH=CH, C=C, ou CR₁₁R₁₂ ;
X représente O, NH, ou S ;
Y représente C, S=O, ou P-OR₁₁ ;
R₄ est choisi dans le groupe comprenant un groupe alkyle de 1 à 10 atomes de carbone, un groupe hydroxyalkyle de 1 à 10 atomes de carbone, un groupe acyloxyalkyle dans lequel le groupement acyloxy est un alkanoyloxy de 2 à 5 atomes de carbone ou un benzoyloxy, et le groupement alkyle contient 1 à 8 atomes de carbone, un groupe benzyle, (phényl)éthyle et phényle, ledit benzyle, (phényl)éthyle ou substituant du phényle étant optionnellement substitué sur l'anneau de benzène par 1 ou 2 groupements sélectionnés indépendamment dans le groupe comprenant un groupe alkyle de 1 à 5 atomes de carbone, un alkoxy de 1 à 5 atomes de carbone et un halogène, à la condition que si ledit anneau de benzène est substitué par 2 desdits groupements, lesdits groupements ne contiennent alors ensemble pas plus de 8 atomes de carbone ;
R₅ est choisi dans le groupe comprenant l'hydrogène, un groupe alkyle de 1 à 10 atomes de carbone, un groupe benzyle, (phényl)éthyle et phényle, ledit benzyle, (phényl)éthyle ou substituant du phényle étant optionnellement substitué sur l'anneau de benzène par 1 ou 2 groupements sélectionnés indépendamment dans le groupe comprenant un groupe alkyle de 1 à 5 atomes de carbone, un alkoxy de 1 à 5 atomes de carbone et un halogène, à la condition que si ledit anneau de benzène est substitué par 2 desdits groupements, lesdits groupements ne contiennent alors ensemble pas plus de 8 atomes de carbone ;
R₆ est choisi dans le groupe comprenant l'hydrogène, un groupe alkyle de 1 à 5 atomes de carbone, et un alkoxy de 1 à 5 atomes de carbone et un halogène ;
R₇ est choisi dans le groupe comprenant l'hydrogène, un groupe alkyle de 1 à 5 atomes de carbone, et un alkoxy de 1 à 5 atomes de carbone et un halogène ;
R₈ représente une chaîne ramifiée ou droite, -(CH₂)ₙ-, où a= 0, 1, 2, 3, 4, 5, 6, 7, 8, 9, 10, dans -(CH₂)ₙ, tout CH₂ peut être remplacé par O, S, CH=CH, C≡C, ou CR₁₁R₁₂ ;
R₉ représente une chaîne ramifiée ou droite, -(CH₂)_{b}-, où b= 0, 1, 2, 3, 4, 5, 6, 7, 8, 9, 10, dans -(CH₂)_{b}-, tout CH₂ peut être remplacé par O, S, CH=CH, C≡C, ou CR₁₁R₁₂ ;
R₁₀ représente une chaîne ramifiée ou droite, -(CH₂)_{c}-, où c- 0, 1, 2, 3, 4, 5, 6, 7, 8, 9, 10, dans -(CH₂)_{c}-, tout CH₂ peut être remplacé par O, S, CH=CH, C≡C, ou CR₁₁R₁₂ ;
R₁₁ représente H, un de tous résidus d'alkyle, alkyloxy, alkényle, perfluoroalkyle, halogénure d'alkyle, ou alkynyle ayant jusqu'à 15 atomes de carbone, des groupements aryle ou hétéroaryle, où tout CH₂ peut être remplacé par O, S, CH=CH, C≡C, ou CR₁R₂ ;
R₁₂ représente H, un de tous résidus d'alkyle, alkyloxy, alkényle, perfluoroalkyle, halogénure d'alkyle ou alkynyle ayant jusqu'à 15 atomes de carbone, des groupements aryle ou hétéroaryle, où tout CH2 peut être remplacé par O, S, CH=CH, C≡C, ou CR₁R₂ ;
A⁻ représente un ion négatif ;
et tous les groupes R, Rₜ, R₂, R₃, R₄, , -(CH₂)ₙ-, -(CH₃)ₘ-, -(CH₂)ₐ-, -(CH₂)_{b}-, ou (CH₂)_{c}-sont des chaînes ramifiées ou droites et peuvent comprendre des atomes C, H, O, S, P, ou N et peuvent avoir des liaisons simples, doubles et triples.

2. Composé choisi dans le groupe comprenant : sarcosine 1-isobutyl-1H-imidazo[4, 5-c] quinolin-4-amide.HA, N,N- diméthylglycine 1-isobutyl-1H-imidazo[4, 5-c]quinolin-4-amide.HA, sarcosine 1-butyl- 1H-imidazo[4, 5-c]quinolin-4-amide.HA, sarcosine 1-benzyl-1H-imidazo [4, 5-c]quinolin- 4-amide.HA, sarcosine 1-méthyl- H-imidazo [4, 5-c] quinolin-4-amide.HA, sarcosine 1, 2, 8-triméthyl-1H-imidazo[4, 5-c]quinolin-4-amide,HA, sarcosine 1-(2-hydroxyéthyl)-1H- imidazo[4,5-c]quinolin-4-amide.HA, sarcosine 1, 8-diméthyl-1H-imidazo[4, 5-c]quinolin- 4-amide.HA, sarcosine 1, 2-diméthyl-1H-imidazo[4, 5-c]quinolin-4-amide.HA, sarcosine 1-(2,3-dihydroxypropyl)-1H-imidazo[4, 5-c]quinolin-4-amide.HA, sarcosine 1- cyclohexylméthyl-1H-imidazo[4, 5-c]quinolin-4-amide.HA, sarcosine 1-benzyl-2-méthyl-1H-imidazo[4,5-c]quinolin-4-amide.HA, sarcosine 1-n-hexyl-1H-imidazo[4, 5-c]quinolin-4-amide.HA, et sarcosine 1-n-hexyl-2-méthyl-1H-imidazo[4,5-c]quinolin-4-amide.HA, où A⁻ représente un ion négatif.

3. Procédés pour la préparation d'un composé ayant une formule générale selon la Structure 1 ou la Structure 2 conformément à la revendication 1 ou d'un composé selon la revendication 2, dans lesquels le composé est préparé à partir de 1H-imidazo[4, 5-c]quinolin-4-amines ou de composés connexes, par réaction avec un composé ayant une formule générale selon la structure 3 en utilisant un réactif de couplage choisi dans le groupe comprenant : N,N'-dicyclohexylcarbodiimide, N,N'-diisopropylcarbodiimide, O-(benzotriazol-1- -yl)-N,N,N',N'-tétraméthyluronium tétrafluoroborate, O-(benzotriazol-1-yl)-N,N,N',N'-tétraméthyluronium hexafluorophosphate, et benzotriazol-1-yl-oxy-tris(diméthylamino)phosphonium hexafluorophosphate, 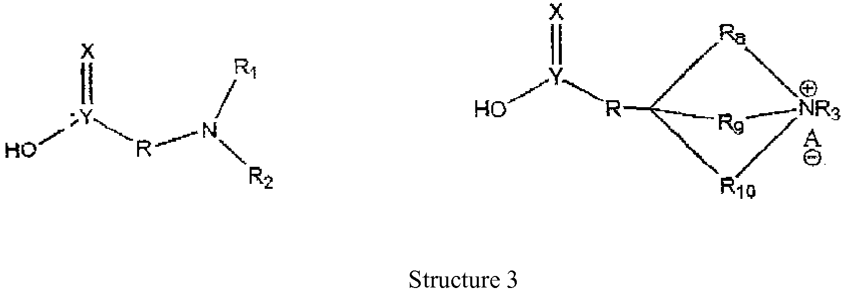
où R représente une chaîne ramifiée ou droite, -(CH₂)ₙ-, où n= 0, 1, 2, 3, 4, 5, 6, 7, 8, 9, 10, dans -(CH₂)ₙ-, tout CH₂ peut être remplacé par O, S, CH-CH, OC, CR₁₁R₁₂, résidus d'aryle ou d'hétéroaryle ;
R₁ et R₂ pris indépendamment sont identiques ou différents et sont H, un de tous résidus d'alkyle, alkyloxy, alkényle ou alkynyle ayant jusqu'à 15 atomes de carbone, des groupements aryle ou hétéroaryle ou pris ensemble sont -(CH₂)ₘ-, où m= 2, 3, 4, 5, 6, 7, 8, 9, 10, et tout CH peut être remplacé par O, S, CH=CH, C=C, des groupements aryle ou hétéroaryle ;
X représente O, NH, ou S ;
Y représente C, S=O, ou P-OR₁₁;
R₃ représente H, un de tous résidus d'alkyle, alkyloxy, alkényle ou alkynyle ayant jusqu'à 15 atomes de carbone, des groupements aryle ou hétéroaryle ;
R₈ représente une chaîne ramifiée ou droite, -(CH₂)ₐ, où a= 0, 1, 2, 3, 4, 5, 6, 7, 8, 9, 10, dans -(CH₂)ₐ-, tout CH₂ peut être remplacé par O, S, CH=CH, C≡C, CR₁R₂, résidus d'aryle ou d'hétéroaryle ;
R₉ représente une chaîne ramifiée ou droite, -(CH₂)_{b}-, où b= 0, 1, 2, 3, 4, 5, 6, 7, 8, 9, 10, dans -(CH₂)_{b}-, tout CH₂ peut être remplacé par O, S, CH=CH, C≡C, CR₁₁R₁₂, résidus d'aryle ou d'hétéroaryle ;
R₁₀ représente une chaîne ramifiée ou droite, -(CH₂)_{c}-, où c= 0, 1, 2, 3, 4, 5, 6, 7, 8, 9, 10, dans -(CH₂)_{c}-, tout CH₂ peut être remplacé par O, S, CH=CH, C≡C, CR₁₁R₁₂, résidus d'aryle ou d'hétéroaryle ;
R₁₁ représente H, un de tous résidus d'alkyle, alkyloxy, alkényle, perfluoroalkyle, halogénure d'alkyle ou alkynyle ayant juqu'à 15 atomes de carbone, des groupements aryle ou hétéroaryle, où tout CH₂ peut être remplacé par O, S, CH=CH, C≡C, CR₁R₂, des groupements aryle ou hétéroaryle ;
R12 représente H, un de tous résidus d'alkyle, alkyloxy, alkényle, perfluoroalkyle, halogénure d'alkyle ou alkynyle ayant jusqu'à 15 atomes de carbone, des groupements aryle ou hétéroaryle, où tout CH₂ peut être remplacé par O, S, CH=CH, C≡C, CR₁R₂-, des groupements aryle ou hétéroaryle ;
A⁻ représente un ion négatif ;
et tous les groupes R, R₁, R₂, R₃, R₄, -(CH₂)n-, -(CH₂)ₘ-, -(CH₂)ₐ-, -(CH₂)_{b}-, ou -(CH₂)_{c}-sont des chaînes ramifiées ou droites et peuvent comprendre des atomes C, H, O, S, P ou N et peuvent avoir des liaisons simples, doubles ou triples.

4. Composé selon l'une quelconque des revendications 1 à 3, dans lequel A représente Cl⁻, Br⁻, F⁻, I⁻, AcO⁻ ou un citrate.

5. Composé selon la revendication 1 ou la revendication 2, ou composition comprenant au moins un composé selon la revendication 1 ou 2, devant être utilisé dans le traitement d'une maladie traitable par 1H-imidazo[4, 5-c]quinolin-4-amines ou des composés connexes chez les humains ou les animaux, la maladie traitable par 1H-imidaze[4, 5-c]quinolin-4-amines ou composés connexes étant choisie dans le groupe comprenant : syndrome d'immunodéficience acquise (SIDA), grippe aviaire, hydrophobie (infection par la rage), verrues, syndrome respiratoire aigu sévère (SRAS), eczéma, hépatite, psoriasis, sclérose en plaques, thrombocytémie essentielle, maladies virales, kératose actinique, carcinome basocellulaire, cancers de la peau, cancer du sein, cancer du côlon, cancer du poumon, cancers de la bouche et cancers, et le composé ou la composition étant administré par voie orale ou transdermique.

6. Composé ou composition devant être utilisé selon la revendication 5, le composé ou la composition étant sous forme de solution, pulvérisation, lotion, onguent, émulsion ou gel, et le composé ou la composition étant administré par voie transdermique.

7. Composé selon la revendication 1 ou la revendication 2, ou composition comprenant au moins un composé selon la revendication 1 ou la revendication 2 comme principe actif devant être utilisé dans le traitement ou la prévention du syndrome d'immunodéficience acquise (SIDA), le composé ou la composition étant sous forme de solution, pulvérisation, lotion, onguent, émulsion ou gel, le composé selon la revendication 1 ou la revendication 2 étant employé à des niveaux thérapeutiques efficaces et le composé ou la composition étant administré par voie transdermique dans les voies respiratoires supérieures et/ou la région génitale d'un sujet humain de sexe masculin ou féminin.

8. Condom devant être utilisé pour la prévention du syndrome d'immunodéficience acquise (SIDA), le condom comprenant
(1) des matières de condom mélangées à un composé selon la revendication 1 ou la revendication 2 ou à une composition comprenant au moins un composé selon la revendication 1 ou la revendication 2, ou
(2) un revêtement comprenant un composé selon la revendication 1 ou la revendication 2 ou une composition comprenant au moins un composé selon la revendication 1 ou la revendication 2, dans lequel le composé ou la composition joue le rôle de principe actif, des niveaux thérapeutiques efficaces d'un composé selon la revendication 1 ou la revendication 2 sont employés, et le revêtement est sous forme de solution, pulvérisation, lotion, onguent, émulsion ou gel.

9. Systèmes d'application thérapeutique transdermique d'un composé selon la revendication 1 ou la revendication 2 devant être utilisés dans le traitement du syndrome d'immunodéficience acquise (SIDA), de la grippe aviaire, de l'hydrophobie (infection par la rage), des verrues, du syndrome respiratoire aigu sévère (SRAS), de l'eczéma, de l'hépatite, du psoriasis, de la sclérose en plaques, de la thrombocytémie essentielle, des maladies virales, de la kératose actinique, du carcinome basocellulaire, des cancers de la peau, du cancer du sein, du cancer du côlon, du cancer du poumon, des cancers de la bouche et des cancers.

10. Systèmes d'application thérapeutique transdermique selon la revendication 9, les systèmes comprenant un bandage ou un patch constitué d'une couche matricielle contenant un principe actif et d'une couche de renfort imperméable.

11. Systèmes d'application thérapeutique transdermique selon la revendication 9, les systèmes comprenant un réservoir de principe actif qui a un fond perméable face à la peau, où grâce à la régulation du débit d'administration, ce système permet aux 1H-imidazo[4, 5- c] quinolin-4-amines et aux composés connexes d'atteindre des niveaux sanguins thérapeutiques constamment optimaux pour augmenter l'efficacité et réduire les effets secondaires des 1H-imidazo[4, 5- c]quinolin-4-amines et composés connexes.

12. Composé selon la revendication 1 ou la revendication 2, ou composition comprenant au moins un composé selon la revendication 1 ou la revendication 2, devant être utilisé dans le traitement du cancer, le composé ou la composition étant en outre combiné à une deuxième composition, la deuxième composition étant sélectionnée dans le groupe comprenant des médicaments anti-inflammatoires non stéroïdiens, des promédicaments de ceux-ci, diéthylaminoéthyl 2-(pisobutylphényl) propionate.AcOH, diéthylaminoéthyl 1-(pchlorobenzoyl)-5-méthoxy-2-méthylindole 3- acétate.AcOH, diéthylaminoéthyl (Z)-5-fluoro-2-méthyl-1-[(4-méthylsulfinyl) phénylméthylène]-1H-indene-3-acétate.AcOH, diéthylaminoéthyl 1-méthyl-5-(4- méthylbenzoyl)-1H-pyrrole-2-acétate.AcOH, diéthylaminoéthyl 5-(4-Chlorobenzoyl)-1,4- diméthyl-1Hpyrrole-2-acétate.AcOH, et diéthylaminoéthyl 1,8-diéthyl-1,3,4,9- tétrahydropyrano-[3,4-b]indolel-acétate.AcOH.

13. Composé selon la revendication 1 ou la revendication 2, ou composition comprenant au moins un composé selon la revendication 1 ou la revendication 2 comme principe actif devant être utilisé dans le traitement du cancer, le composé ou la composition étant administré par voie orale ou transdermique avant et/ou après ablation chirurgicale d'une tumeur.
